# EUROPEAN PATENT APPLICATION

(11) **EP 1 903 028 A1**
(43) Date of publication of application: **26.03.2008**
(21) Application number: 06019890.0
(22) Date of filing: 22.09.2006
(51) Int. Cl.: C07C 237/00, C07C 237/24, A61K 31/198, A61P 25/28

(54) **Conjugates of glutamate and NMDA receptor channel blockers without excito-toxic effects**

(71) Applicant: Tschollar,, Werner, 1197 Prangins (CH); Lanser,, Marc Eric, Fayston VT 05673 (US)
(72) Inventor: Tschollar,, Werner, 1197 Prangins (CH); Lanser,, Marc Eric, Fayston VT 05673 (US)
(74) Representative: Jones Day

(57) **Abstract**

Methods for delivering therapeutic amounts of glutamate to the CNS while simultaneously protecting against its excito-toxic effects are provided. By administering a blood-brain barrier-permeable Conjugate Compound, which comprises glutamate linked to an N-methyl-D-aspartate receptor antagonist, glutamate is released within the CNS for beneficial effect without causing excito-toxicity. Examples of such Conjugate Compounds are provided and their cognition-enhancing effect without engendering excito-toxicity in both normal and cognition-impaired animals is demonstrated.

## Description

### FIELD OF THE INVENTION

The present invention relates to Conjugate Compounds, pharmaceutical compositions comprising effective amounts of Conjugate Compounds, methods of their use for enhancing learning, memory, and cognition, methods for treating and preventing mood disorders, methods for treating or preventing neurodegenerative disorders, and methods useful for treating or preventing disorders involving cognitive impairment, such as schizophrenia, Alzheimer's disease, and attention deficit hyperactivity disorder. The Conjugate Compounds are also useful for preventing neuronal cell death. The Conjugate Compounds are also useful for enhancing cognitive activity while protecting neurons from excito-toxicity, and for neuro-protection while avoiding cognitive impairment.

### BACKGROUND OF THE INVENTION

Glutamate is a neurotransmitter within the Central Nervous System (CNS). There are 4 different subtypes of glutamate receptors: metabotropic receptors coupled to G-proteins; and the ligand-gated ionotropic receptors: alpha-amino-3-hydroxy-5-methyl-4-isoxazolepropionic acid (AMPA) receptors, kainate receptors, and the N-methyl-D-aspartate (NMDA) receptor. The NMDA receptor (NMDAR) is believed to be the only glutamate receptor subtype that is permeable to Ca and to play a major role in mediating the excito-toxic effects in the CNS of exposure to persistent, excess glutamate.

Glutamate is well-known to play important functions in learning, memory, cognition and mood. Specifically, timed NMDA receptor activation contributes to long term potentiation (LTP) in the hippocampus, which is believed to be the physiologic basis for memory formation. The negative symptoms of schizophrenia (cognitive impairment, anhedonia, apathy, social withdrawal) are currently believed to be caused by a so-called "hypo-glutamatergic state", since experimentally, NMDA blockade results in symptoms that closely resemble the cognitive impairment of schizophrenia in animals, and the NMDA channel-blocking drugs ketamine and phencyclidine (PCP) engender a schizophrenic-like state in humans. The negative symptoms of schizophrenia respond to NMDA agonists directed at the glycine co-agonist site, such as glycine, serine, and cycloserine, though direct agonist therapy has not as yet been practical.

In addition to schizophrenia, hypo-function of the glutamatergic system has been hypothesized to be responsible for the cognitive decline in Alzheimer's disease (AD), as well as the cognitive dysfunction in Attention Deficit Hyperactivity Disorder (ADHD).

The physiologic role and therapeutic importance of the other glutamate receptors are less well defined. Though metabotropic receptors serve important physiologic processes independently, they also serve as modulators of NMDA receptor activity. Experimental data suggests a positive modulatory role for group I metabotropic receptors (mGlu1 and mGlu5) on NMDA receptor function, while group II (mGlu2 and mGlu3) activation appears to negatively modulate NMDA receptor function. AMPA and kainate receptors also have independent and inter-related effects on other glutamatergic receptors, including NMDA receptors.

Though potentially beneficial, direct systemic glutamate therapy for cognitive impairment remains impractical because of the inability of glutamate to cross the blood-brain barrier (BBB) to any appreciable extent, and because of the risk of engendering glutamate-induced excito-toxicity. Though glutamate has relatively low affinity for the NMDA receptor (EC₅₀ approx. 10microM), unregulated and persistent stimulation of NMDA receptors by glutamate leads to excessive calcium influx and eventual apoptotic neuronal cell death.

The present invention provides for the simultaneous partial blockade of NMDA receptors, while still allowing for glutamate-activation of these and the non-NMDA receptors. Systemic administration of a compound of the present invention to animals results in cognitive enhancement without engendering excito-toxicity. Because glutamate accomplishes its cognition-enhancing effects through activation of the NMDAR, the positive effects of concurrent administration of glutamate and an NMDA channel blockers were unexpected.

Neboglamine for enhancing memory and learning, and as neuroprotective agent have been described in U.S. Patent No. 5,723,494 issued March 3, 1998 and in PCT application no. PCT/EP2005/052340 published as WO 2005/115373. Both of which are incorporated herein by reference in their entireties.

### SUMMARY OF THE INVENTION

In certain embodiments, the present invention encompasses compounds of Formula (I): 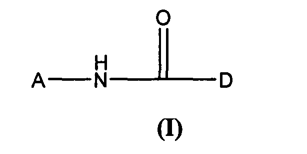 and pharmaceutically acceptable salts thereof, wherein:
A is an NMDA channel blocker; and
D is a Glutamate analog or 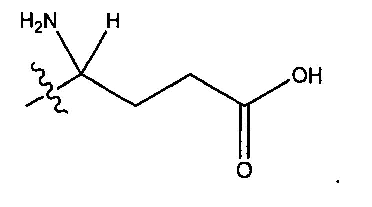
Compounds of Formula I will be referred to as Conjugate Compounds.

In certain embodiments, Conjugate Compounds of the invention are compounds of Formula I with the proviso that the Conjugate Compound is not:

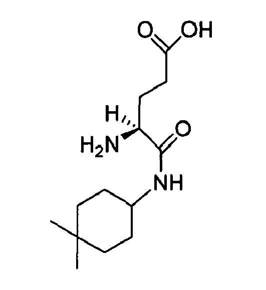

### Compound 1

### (S)-4-amino-5-(4,4-dimethylcyclohexylamino)-5-oxopentanoic acid

In certain specific embodiments, the NMDA channel blocker is neramexane (1,3,3,5,5-pentamethylcyclohexanamine). Thus, a resulting Conjugate Compound of the invention is:

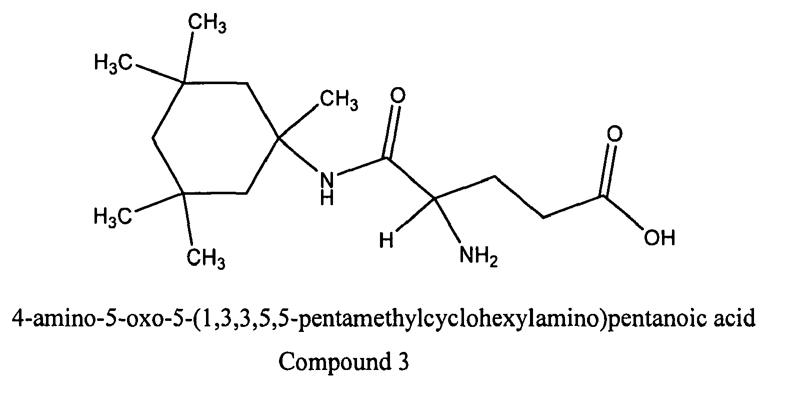

In certain specific embodiments, the NMDA channel blocker is memantine (3,5-dimethyladamantan-1-amine). Thus, a resulting Conjugate Compound of the invention is:

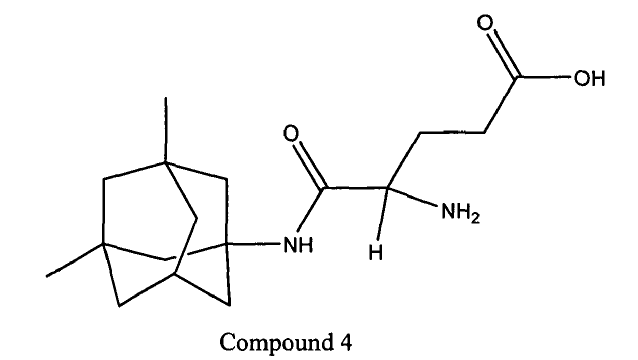

In certain embodiments, the invention also provides Conjugate Compounds comprising an NMDA channel blocker linked to glutamate via a bond that is cleavable by an enzyme within the CNS. In certain embodiments, the bond is a peptide bond. In specific embodiments, the bond can be cleaved by aminopeptidase A.

In certain embodiments, the invention provides a composition comprising an effective amount of a Conjugate Compound and a pharmaceutically acceptable carrier, excipient, or solvent. In certain embodiments, the Conjugate Compound is not Compound 1.

In certain embodiments, the present invention provides methods for treating a mammal, including a human, simultaneously with glutamate and an NMDA channel blocker comprising a Conjugate Compound. Such treatment allows for the administration of glutamate without inducing excito-toxicity, for the treatment of conditions associated with cognitive impairment. In specific embodiments, the condition associated with cognitive impairment is schizophrenia, Alzheimer's disease, or attention deficit hyperactivity disorder. In certain embodiments, the Conjugate Compound is not Compound 1.

In a further embodiment of the invention, the Conjugate Compound according to the present invention is used to treat conditions believed to benefit from neuro-protection, and where further cognitive impairment is to be avoided, as in the treatment of stroke, Alzheimer's disease, vascular dementia, traumatic brain injury, Parkinson's disease, and other neurodegenerative diseases. In certain embodiments, the Conjugate Compound is not Compound 1.

In a further embodiment of the invention, the Conjugate Compound is used to treat defects in cognition by increasing glutamatergic activity without engendering excito-toxicity, as in the treatment of Alzheimer's disease, schizophrenia, autism, depression, and ADHD. In certain embodiments, the Conjugate Compound is not Compound 1.

In a further embodiment of the invention, the Conjugate Compound is used to treat conditions believed to benefit from partial NMDA channel blockade, and where further cognitive impairment is to be avoided, as in the treatment of drug addiction (including smoking), depression, anxiety, neuropathic pain, and alcohol abuse. In certain embodiments, the Conjugate Compound is not Compound 1.

Acid-addition and/or base salts of the Conjugate Compounds are within the scope of the present invention.

### BRIEF DESCRIPTION OF THE DRAWINGS

FIG. 1 shows the penetration into the CNS of the rat of Compound 1 after a single oral administration.
FIG. 2 shows the cleavage of Compound 1 into the Compound 2 plus glutamate by rat hippocampal slices *in vitro.* The cleavage is inhibited by the specific aminopeptidase A inhibitor amastatin. The amount of Compound 2 produced by cleavage of Compound 1 after 3 hours incubation with hippocampal slices is shown. AMA=Amastatin.
FIGS. 3A,B show inhibition of NMDA function by Compound 2.

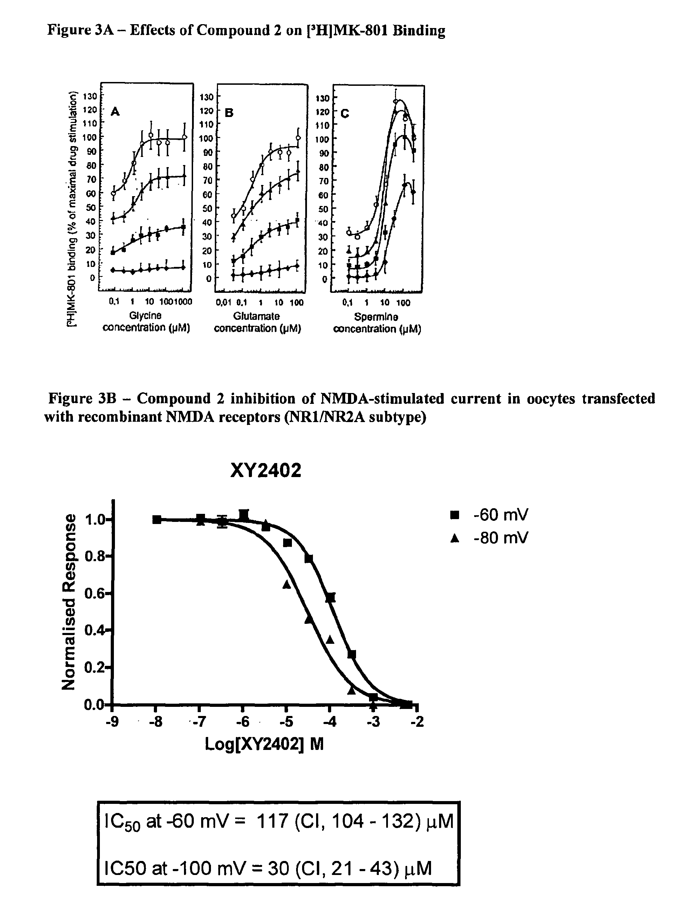

   FIG. 3A shows the inhibition by Compound 2 on MK801 binding to the open channel of the NMDA receptor. Effects of Compound 2 on [³H]MK-801 binding enhanced by glycine (panel A), glutamate (panel B) and spermine (panel C). The stimulation of [³H]MK-801 binding as a percent of stimulation induced by the maximal agonist concentration is shown in the absence (o) and presence of 10µM (▲), 100µM (■) and 1000µM (◆) Compound 2. The 3H-MK-801 concentration was approx. 3 nM in all the experiments.
   FIG. 3B shows voltage-dependant inhibition by Compound 2 of glutamate-evoked currents in NMDA receptor-transfected oocytes

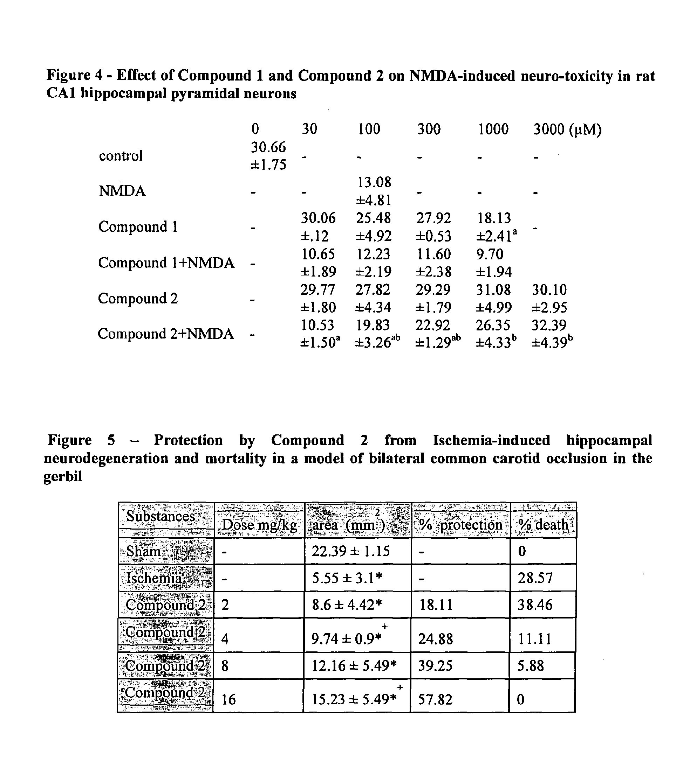

FIG. 4 shows the neuro-protective effect of Compound 2 against NMDA excito-toxicity *in vitro.* Values (area mm²) represent mean ± SD of three series of experiments. a: significantly different (p<0.05) from control slices b: significantly different (p<0.05) from NMDA-treated hippocampal slices.

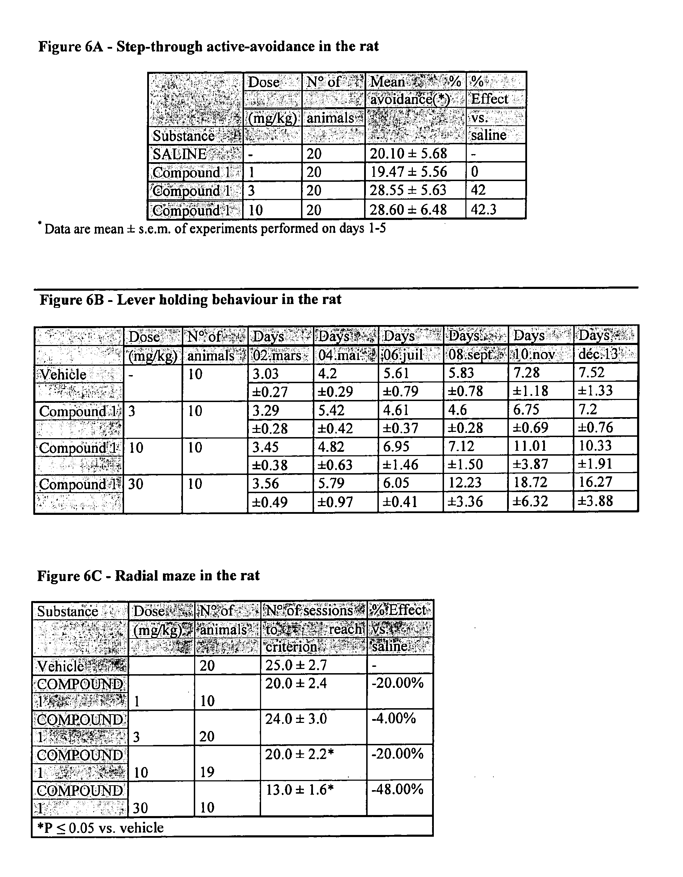

FIG. 5 shows the neuro-protective effect of Compound 2 against bilateral carotid artery occlusion *in vivo.* Data are mean ± SD. * P <0.05 vs. sham animals. + P <0.05 vs. ischemic animals.
FIGS. 6A-C show the cognitive-enhancing effect of Compound 1 in normal animals
   FIG. 6A shows the effect of Compound 1 on the step-through active avoidance test in rats. Effect of Compound 1 administered i.p. twice a day (30 min before session and at 5.00 p.m.) for 4 days. The fifth day Compound 1 was administered 30 min before session. * Data are mean ± s.e.m. of experiments performed on days 1-5
   FIG. 6B shows the effect of Compound 1 on operant-conditioned lever-holding behavior in rats. Data are Mean ± s.e.m. of the efficiency indexes grouped in 2-day intervals.
   FIG. 6C shows the effect of Compound 1 on learning behavior in the radial maze in rats.

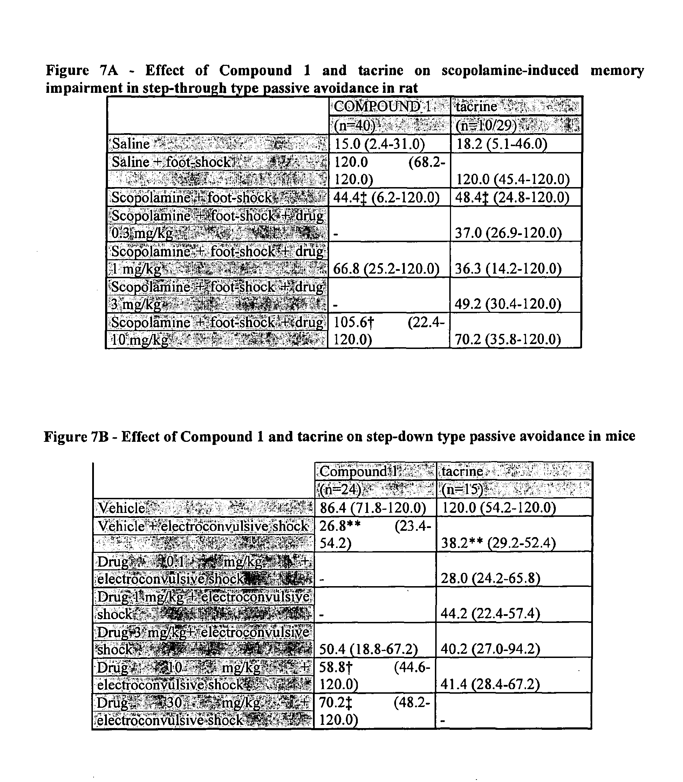

FIGS. 7A,B show the cognitive-enhancing effect of Compound 1 in impaired animals
   FIG. 7A shows the effect of Compound 1 on scopolamine-induced memory impairment. Values represent the median latency (sec) to enter the shock compartment and interquartile ranges. †P <0.05 vs. scopolamine + (FS), ‡P < 0.01 vs. saline + (FS) (log rank test).
   FIG. 7B shows the effect of Compound 1 on electroconvulsive shock-induced memory impairment. Values represent the median latency (s) to enter the shock compartment and interquartile ranges. **P < 0.01 vs. vehicle no ECS, †P< 0.05 and ‡P< 0.01 vs. vehicle + ECS (log rank test).

### DEFINITIONS, CONVENTIONS, AND ABBREVIATIONS

As used herein, the term "Conjugate Compound" refers to a compound of Formula 1. In certain embodiments, the Conjugate Compound is not Compound 1.

It is recognized that the Conjugate Compounds can have one or more chiral centers and/or double bonds and, therefore, exist as stereoisomers, such as double-bond isomers (i.e., geometric isomers), enantiomers, or diastereomers. According to the invention, the chemical structures depicted herein, and therefore the compounds of the invention, encompass all of the corresponding enantiomers and stereoisomers, that is, both the stereomerically pure form (e.g., geometrically pure, enantiomerically pure, or diastereomerically pure) and enantiomeric and stereoisomeric mixtures, e.g., racemates.

As used herein, an "effective amount" when used in connection with a Conjugate Compound refers to that amount of the Conjugate Compound useful for treating or preventing of schizophrenia, Alzheimer's Disease, Attention Deficit Hyperactivity Disorder, drug addiction, depression, anxiety, neuropathic pain, alcohol abuse, autism, depression, stroke, vascular dementia, traumatic brain injury, or Parkinson's disease. In particular, the term "effective amount" refers to that amount of a Conjugate Compound that ameliorates one or more symptoms of any one of these conditions. The term "effective amount" also refers to that amount of a Conjugate Compound that increases the congnitive abilities of a mammal.

As used herein, the terms "cognition and cognitive impairment" in the context of experimental studies, refer to the observable behavioral tasks of learning and memory and impairment on those tasks in animals , respectively.

As used herein, the terms "excitotoxicity" and "neuro-toxicity" are used interchangeably to denote the pathologic process of glutamate-induced apoptotic neuronal cell death that is mediated by glutamate activation of the NMDA receptor.

As used herein, the language "NMDA channel-blocker" is art recognized and is intended to include any NMDA receptor antagonist that binds to the ion channel of the NMDA receptor and inhibits the cellular influx of Ca through that channel.

As used herein, the language "NMDA receptor antagonist" is art recognized and is intended to include any known or anticipated NMDA receptor antagonist that binds to any site on the NMDA receptor and has a direct or indirect inhibitory effect on NMDA receptor activity, including (but not limited to) the Ca channel, the glycine-site, the glutamate site, or the ifenprodil site.

As used herein, the language "linked" is art recognized and is intended to include any chemical bond between the NMDA antagonist and glutamate that is susceptible to enzymatic cleavage.

**Abbreviation**

| | |
|---|---|
| BBB | Blood-brain barrier |
| AMPA | alpha-amino-3-hydroxy-5-methyl-4-isoxazolepropionic acid |
| NMDA | N-methyl-D-aspartate |
| AD | Alzheimer's Disease |
| CNS | Central Nervous System |
| Cbz | Carbobenzoxy |

### DETAILED DESCRIPTION OF THE INVENTION

In certain embodiments, the present invention provides compounds, pharmaceutical compositions, and methods for the therapeutic administration of glutamate without engendering glutamate-induced excito-toxicity. Conversely, the present invention also provides compounds, pharmaceutical compositions, and methods for therapeutic neuro-protection without impairing cognition. Without being bound by theory, the invention is based, at least in part, on the discovery that administration of a Conjugate Compound comprising an NMDA channel-blocker linked to glutamate provides effective cognitive enhancement in normal and impaired animals without neurotoxicity (see Examples VII to IX).

NMDA channel blockers are potent neuro-protective agents but their use is limited by their negative effects on cognition. Similarly, glutamate is known for its cognition-promoting effects, but its use is limited by its excitotoxic effects. The present Conjugate Compounds can provide the benefit of the neuroprotection of an NMDA channel blocker simultaneously with the beneficial effects of glutamate on cognition. Thus, in situations where neuro-protective therapy is beneficial (e.g., stroke, AD, and brain injury), this can be achieved without the risk of cognitive impairment. And vice versa, using the Conjugate Compounds an increase in cognitive abilities can be accomplished while avoiding neurotoxicity.

### Formula I

In certain embodiments, the present invention encompasses compounds of Formula (I): 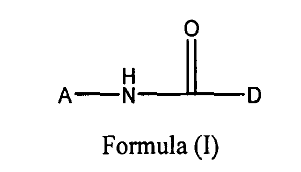 and pharmaceutically acceptable salts and solvates thereof, wherein:
A is an NMDA channel blocker; and
D is a Glutamate analog or 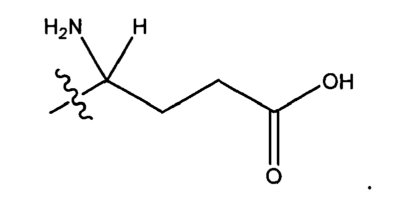
Compounds of Formula (I) will be referred to as Conjugate Compounds.

In certain embodiments, Conjugate Compounds of the invention are compounds of Formula I with the proviso that the Conjugate Compound is not: 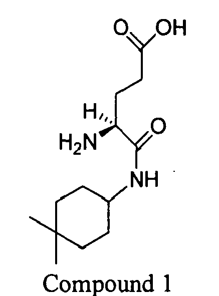 (S)-4-amino-5-(4,4-dimethylcyclohexylamino)-5-oxopentanoic acid

In certain embodiments, the NMDA channel blocker moiety in Formula I is selected from the group consisting of D(-)-2-amino-4-phosphonobutyric acid, D(-)-2-amino-7-phosphonoheptanoic acid, D(-)-2-amino-5-phosphonopentanoic acid, DL-2-amino-5-phosphonopenta- noic acid, R(-)-3-(2-carboxypiperazin-4-yl)-propyl-1-phosphonic acid, (RS)-3-(2-carboxypiperazin-4-yl)-propyl-1-phosphonic acid, 4-Cl-kynurenine, 7-chloro-kynurenic acid, (-)6-phosphonomethyl-decahydroi- soquinoline-3-carboxylic acid, ACPC, aptiganel, besonprodil, BMY-14802, budipine, CGP-37849, CP-101606, conantokin G, CR-3991, CR-2249, CR-3394, delucemine, dexanabinol, dizocilpine, EAA-090, eliprodil, felbamate, fluorofelbamate, FPL-12495, gacyclidine, gavestinel, glycine, harkoseride, HU-211, ibogaine, ipenoxazone, kaitocephalin, ketamine, L-695902, lanicemine, licostinel, ligustizine, midafotel, milnacipran, neboglamine, nebostinel, neramexane, N'-[2-chloro-5-(methylthio)phenyl]-N- -methyl-N-[3-(methylthio)phenyl]-guanidine, N'-[2-chloro-5-(methylthio)phenyl]-N-methyl-N-[3-[(R)-methylsulfinyl]phenyl]-guanidine, neramexane, orphenadrine, remacemide, RGH-896, RG-13484, RG-13579, RG-1103, Ro-25-6981, selfotel, seratrodast, spermidine, spermine, topiramate, traxoprodil, UK-240255, ZD-9379, .alpha.-amino-2-(2-phosphonoethyl)-cyclo- hexanepropanoic acid, alpha,-amino-4-(phosphonomethyl)-benzeneacetic acid, N(1)-(benzyl)cinnamamidine, and 4-benzyl-4-hydroxy-N-(hydroxyphenox- yalkyl)-piperidine.

In certain embodiments, the NMDA channel blocker moiety in Formula (I) is an NMDA receptor antagonist selected from the compounds listed in Table 1.

**Table 1: Exemplary NMDA channel blockers.**

| Compound | Structure | Reference |
|---|---|---|
| Adamantane [281-23-2] Synonyms: Adamantane; Tricyclo[3.3.1.13,7]decane ; tricyclo[3.3.1.1]decane | 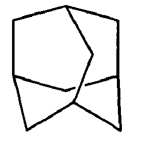 | |
| 5-(2-amino-2-carboxyethyl)-4,5-dihydroisoxazole-3-carboxylic acid | 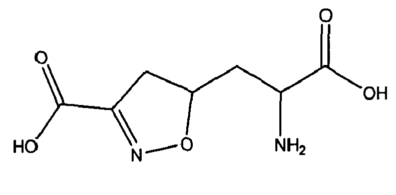 | J Med Chem. 2005 Oct 6;48(20):6315-25 |
| Memantine (3,5-dimethyladamantan-1-amine) | 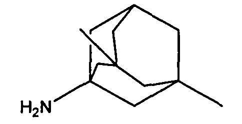 | J Neurochem. 2006 Jun;97(6):1611-26 |
| Cyclohexylbenzene [827-52-1] (Cyclohexylbenzene; Phenylcyclohexane) | 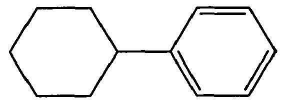 | |
| N-[2-(3,5-dimethyl-1-adamantyl)ethyl] guanidine | 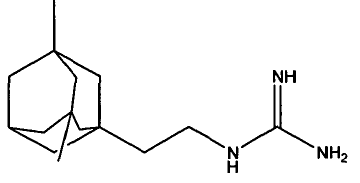 | Neuropharmacology. 2006 Mar;50(3):277-85. Epub 2005 Oct 19 |
| N-[2-(3,5-dimethyl-1-adamantyl) ethyl] acetamidine | 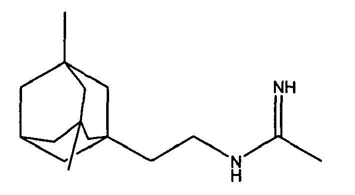 | Neuropharmacology. 2006 Mar;50(3):277-85. Epub 2005 Oct 19 |
| (+)-SKF38393 | 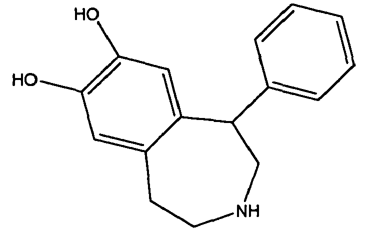 | Br J Pharmacol. 1999 Apr;126(8):1847-55 |
| Dopamine | 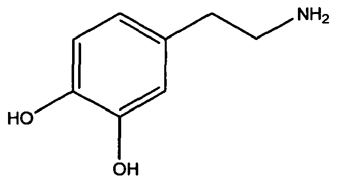 | Br J Pharmacol. 1999 Apr; 126(8): 1847-55 |
| Mono- and dicationic derivatives of adamantane, phenylcyclohexyl, tripphenylmethane, diphenylmethane | | Neuropharmacology. 2005 Aug;49(2): 144-55 |
| amino-alkyl-cyclohexanes | | Neuropharmacology. 1999 Jan;38(1):85-108 |
| Dizocilpine (MK-801), phencyclidine, ketamine, dexoxadrol, remacemide, the | | Neuropharmacology1996;35(12):1709-19. |
| remacemide metabolite 1,2-diphenyl-2-propylamine (ARL 12495), phencylcyclopentylamine, dextromethorphan, (+/-)-5-aminocarbonyl-10,11-dihydro -5H-dibenzo-[a,d]cyclohepten-5,10-imine, levoxadrol | | |
| Imidazolines, such as Antazoline, idazoxan and guanabenz | | Neuropharmacology. 2000 Sep;39(12):2244-54 |
| PD 138289, PD 137889, FR 115427, MRZ 2/579,1-(4-methoxyphenyl)-1,2,3,4-tetrahydroisoquinoline; 8-(2-methoxyphenyl)-1,2,3,4-tetrahydroisoquinoline; alaproclate; phencyclidine | | Psychopharmacolog y (Berl). 2003 Nov;170(2):215-24. Epub 2003 Jul 8 |
| N-alkylglycines, such as 3,3-diphenylpropyl-N-glycinamide (N20C) | | J Pharmacol Exp Ther. 2002 Jul;302(1):163-73 |
| inhibitors of metabotropic glutamate receptors, particularly group I receptors (mGlu 1 and mGlu5) | | |

In certain embodiments, the NMDA channel blocker has one of the following structures: 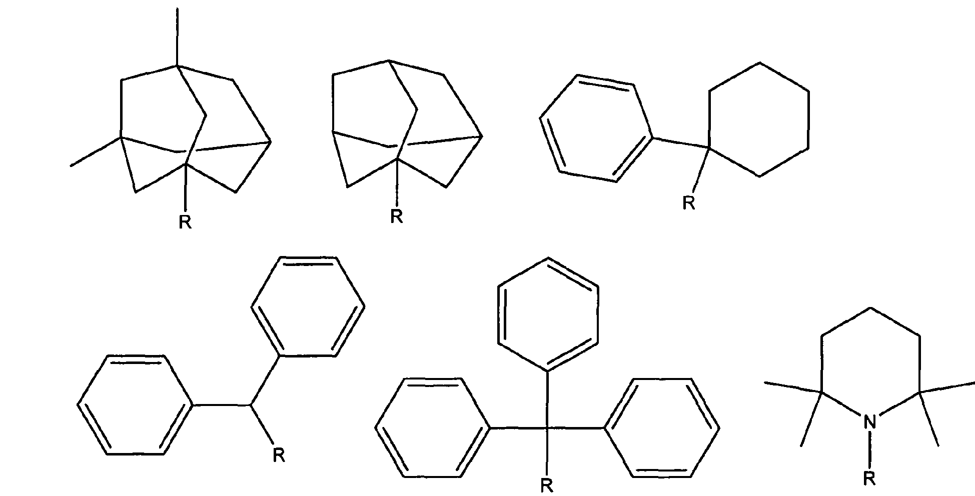 wherein R is C₁ to C₁₀ alkyl or NH-C₁ to C₁₀ alkyl. These NMDA receptor antagonist are linked through R to the remainder of the Conjugate Compound of the invention.

In a preferred embodiment, D in Formula (I) is:

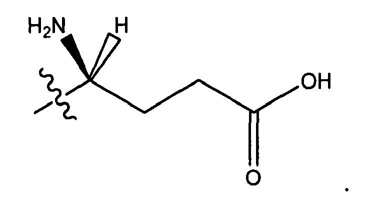

In certain embodiments, hydrolysis of a Conjugate Compound results in an NMDA channel blocker and L-Glutamic acid.

In certain other embodiments, D in Formula (I) is: 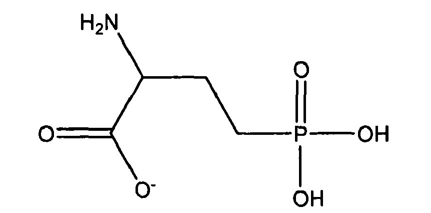 2-amino-4-phosphonobutyrate ; or

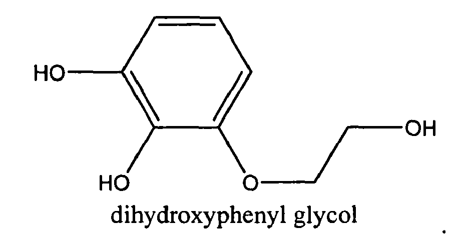

In certain specific embodiments, the NMDA channel blocker, i.e., A in Formula (I) is neramexane (1,3,3,5,5-pentamethylcyclohexanamine), and D in Formula (I) is capable of yielding glutamate upon hydrolysis. Thus, a resulting Conjugate Compound of the invention is:

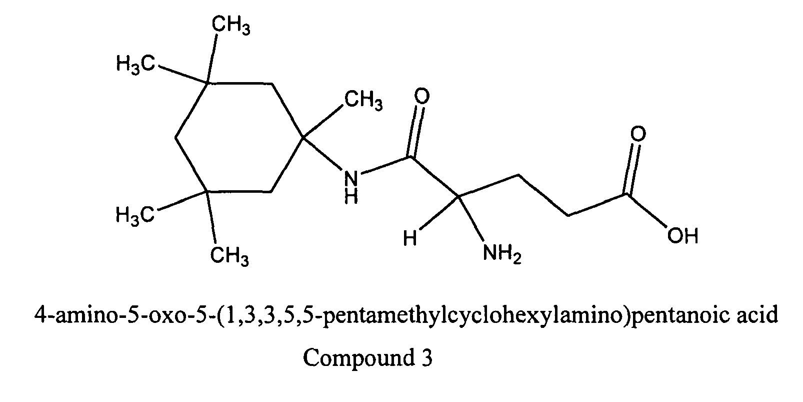

In an even more specific embodiment, the Conjugate Compound of the invention is:

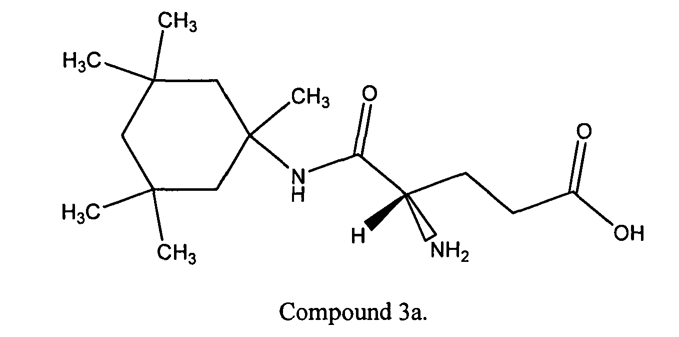

In certain specific embodiments, the NMDA channel blocker, *i.e.,* A in Formula (I) is memantine (3,5-dimethyladamantan-1-amine), and D in Formula (I) is capable of yielding glutamate upon hydrolysis. Thus, a resulting Conjugate Compound of the invention is:

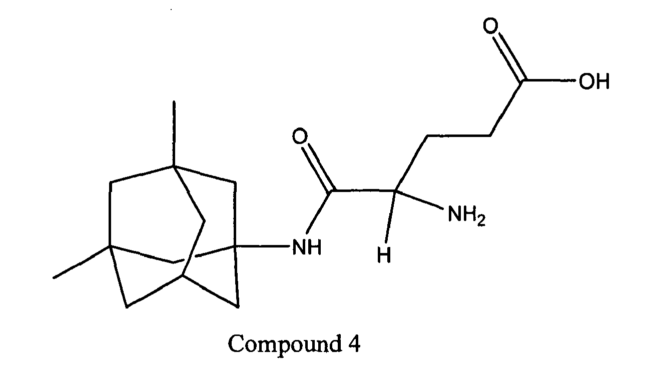

In an even more specific embodiment, the Conjugate Compound of the invention is:

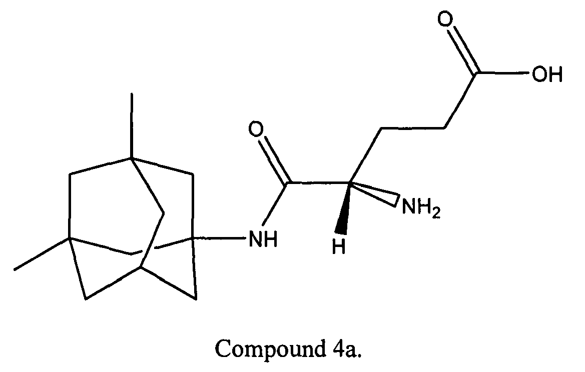

The present invention provides methods for treating or preventing a neurodegenerative disorder, said method comprising administering to a subject an effective amount of a Conjugate Compound. The present invention also provides Conjugate Compounds for curing a neurodegenerative disorder. The present invention also provides the use of a Conjugate Compound for the manufacture of a medicament for curing a neurodegenerative disorder. In a specific embodiment, the neurodegenerative disorder is Alzheimer's disease, vascular dementia, Parkinson's disease, traumatic brain injury. In certain embodiments, the Conjugate Compound is not Compound 1.

The present invention also provides methods for enhancing cognition, said method comprising administering to a subject an effective amount of a Conjugate Compound. The present invention also provides Conjugate Compounds for enhancing cognition. The present invention also provides the use of a Conjugate Compound for the manufacture of a medicament for enhancing cognition. In a specific embodiment, learning and or memory are enhanced. In certain embodiments, the Conjugate Compound is not Compound 1.

The subject can be a human or an animal. In particular, the animal can be a mammal. The mammal can be a dog, cat, horse, monkey, chimpanzee, baboon, cow, sheep, pig, mouse, rat, rabbit, and guinea pig. References to "mammals" include humans.

In certain embodiments, an NMDA channel blocker and glutamate are administered as separate chemical entities, i.e., the NMDA channel blocker and glutamate are not covalently linked. In a specific aspect, NMDA channel blocker and glutamate are administered directly in the CNS, e.g., by using an implant. Thus, the present invention also provides in certain embodiments pharmaceutical formulations and kits in which NMDA channel blocker and glutamate are provided as separate chemical entities. In a specific embodiment, the invention provides an implant comprising a slow release formulation of a combination of NMDA channel blocker and glutamate for implantation into the CNS. For certain indications, where the BBB is interrupted, systemic administration of a combination of an NMDA channel blocker and glutamate are administered as separate chemical entities is also suitable. Thus, in a specific embodiment, a combination of an NMDA channel blocker and glutamate is administered in stroke patients to prevent neurodegeneration while avoiding cognitive impairment.

In a specific embodiment, the invention provides a method for treating or preventing a neurodegenerative disorder, said method comprising administering to a subject in need thereof an effective amount of glutamate and an effective amount of an NMDA channel blocker selected from the group consisting of memantine, neramexane, and 4,4-dimethylcyclohexylamine. In a specific embodiment, the invention provides a method for enhancing cognition, said method comprising administering to a subject an effective amount of glutamate and an effective amount of an NMDA channel blocker selected from the group consisting of memantine, neramexane, and 4,4-dimethylcyclohexylamine.

In certain embodiments, the invention also provides a pharmaceutical composition or an implant comprising an effective amount of glutamate and an effective amount of an NMDA channel blocker selected from the group consisting of memantine, neramexane, and 4,4-dimethylcyclohexylamine.

### Formula II

In certain embodiments, the present invention encompasses compounds of Formula (II): 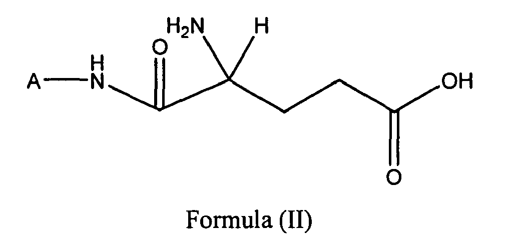 and pharmaceutically acceptable salts and solvates thereof, wherein A is a Group I metabotropic glutamate receptor antagonist or a Group II metabotrobic glutamate receptor agonist. Illustrative Group I metabotropic glutamate receptor antagonists include, but are not limited to, LY3930S3 [(+)-2-amino-2-(3-cis and *trans-*carboxycyclobutyl-3-(9-thioxanthyl)propionic acid], LY367385 [(*S*)-(+)-α-amino-4-carboxy-2-methylbenzeneacetic acid], or AIDA [(R,S)-1-aminoindan- 1,5 -dicarboxylic acid/UPF S23]. An illustrative Group II metabotrobic glutamate receptor agonist is LY3S4740 [1S,2S,5R,6S-2-aminobicydo [3.1.0]hexane-2,6-dicarboxylate monohydrate].

The present invention provides methods for treating or preventing a neurodegenerative disorder, said method comprising administering to a subject an effective amount of a compound of Formula (II). The present invention also provides compounds of Formula (II) for curing a neurodegenerative disorder. The present invention also provides the use of a compound of Formula (II) for the manufacture of a medicament for curing a neurodegenerative disorder.

The present invention also provides methods for enhancing cognition, said method comprising administering to a subject an effective amount of a compound of Formula (II). The present invention also provides compounds of Formula (II) for enhancing cognition. The present invention also provides the use of a compound of Formula (II) for the manufacture of a medicament for enhancing cognition. In a specific embodiment, learning and or memory are enhanced.

### Methods for Making Conjugate Compounds

Conjugate Compounds may be prepared according to the methods which are described therein or by applying methods of chemical synthesis well known to a person skilled in the art. In certain embodiments, the Conjugate Compounds can be prepared by reacting the methyl ester, e.g., of N-carbobenzoxy-L-glutamic acid with the amino group of the NMDA channel blocker or an amine derivative of the NMDA channel blocker.

### ILLUSTRATIVE SYNTHESIS OF COMPOUNDS 3 AND 4

As an illustrative synthesis scheme, the synthesis of Compound 3 is represented in Scheme 1 below. The methyl ester ofN-carbobenzoxy-L-glutamic acid, Compound 8, is reacted with neramexane, Compound 7. Following removal of the carbobenzoxy protective group from resulting Compound 9, Compound 3 is obtained. Neramexane, Compound 7, can be obtained from 3,3,5,5-tetramethylcyclohexanone, Compound 5. Reduction of Compound 5 yields 1,3,3,5,5-pentamethylcyclohexanol, Compound 6, that is transformed to neramexane, Compound 7.

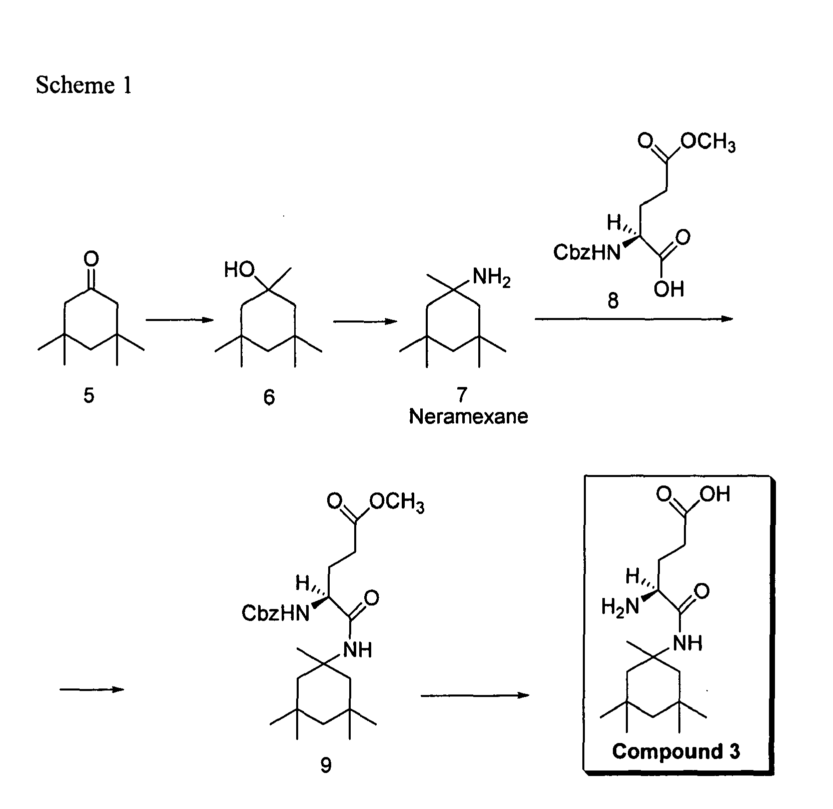

As another illustrative synthesis scheme, the synthesis of Compound 4 is represented in Scheme 2 below. The methyl ester of N-carbobenzoxy-L-glutamic acid, Compound 8, is reacted with memantine, Compound 10. Following removal of the carbobenzoxy protective group from resulting Compound 11, Compound 4 is obtained. Memantine, Compound 10, is a commercially available compound.

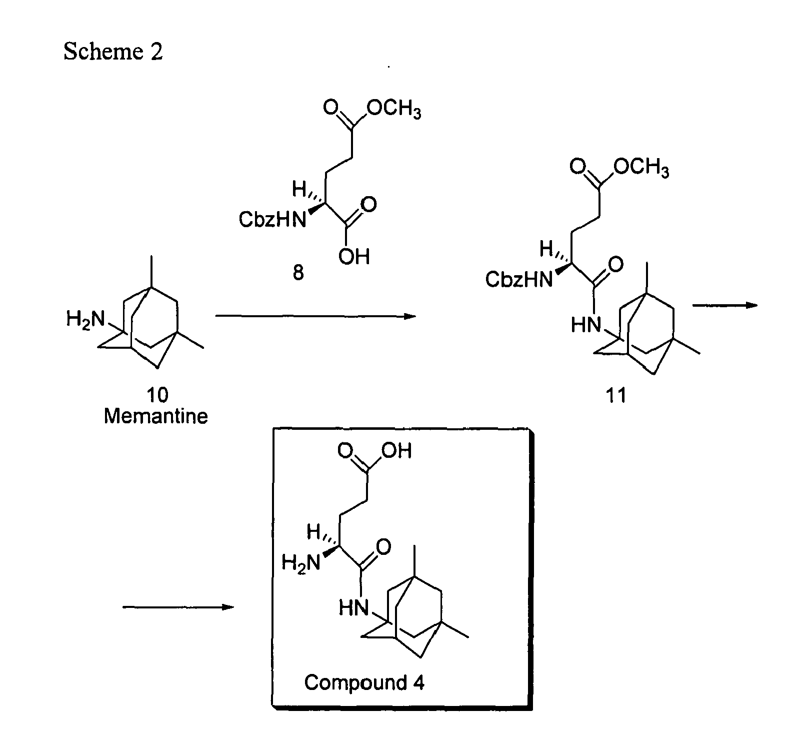

### Therapeutic/Prophylactic Administration And Compositions

In certain embodiments, when administered to a subject, e.g., an animal for veterinary use or to a human for clinical use, or when made to contact a cell or tissue, the Conjugate Compound can be in isolated and purified form.

The present compositions, which comprise a Conjugate Compound, can be administered by any convenient route, for example by infusion or bolus injection, by absorption through epithelial or mucocutaneous linings (e.g., oral mucosa, rectal and intestinal mucosa) and can be administered together with another active agent. Administration can be systemic or local. Various delivery systems are known, e.g., encapsulation in liposomes, microparticles, microcapsules, capsules, etc., and can be used to administer a Conjugate Compound. In certain embodiments, more than one Conjugate Compound is administered to a patient. Methods of administration include but are not limited to intradermal, intramuscular, intraperitoneal, intravenous, subcutaneous, intranasal, epidural, oral, sublingual, intranasal, intracerebral, intravaginal, transdermal, rectally, by inhalation, or topically to the ears, nose, eyes, or skin. The mode of administration can be left to the discretion of the practitioner.

In specific embodiments, the Conjugate Compound is administered by means of an implant, said implant being of a porous, non-porous, or gelatinous material, including membranes, such as sialastic membranes, or fibers. The implantation can be either within the CNS, subcutaneously, or intramuscularly. As one skilled in the art will recognize, such formulation may be achieved by incorporating the Conjugate Compound into microspheres, polymers, or lipids in order to achieve release of the Conjugate Compound over time.

In certain embodiments, it might be desirable to administer one or more Conjugate Compounds by any suitable route, including intraventricular and intrathecal injection. Intraventricular injection can be facilitated by an intraventricular catheter, for example, attached to a reservoir, such as an Ommaya reservoir.

Pulmonary administration can also be employed, e.g., by use of an inhaler or nebulizer, and formulation with an aerosolizing agent, or via perfusion in a fluorocarbon or synthetic pulmonary surfactant. In certain embodiments, the Conjugate Compound can be formulated as a suppository, with traditional binders and carriers such as triglycerides.

In another embodiment, the Conjugate Compound can be delivered in a vesicle, in particular a liposome *(see* Langer, Science 249:1527-1533 (1990); Treat et al., in Liposomes in the Therapy of Infectious Disease and Cancer, Lopez-Berestein and Fidler (eds.), Liss, New York, pp. 353-365 (1989); Lopez-Berestein, *ibid.,* pp. 317-327; *see* generally *ibid.)*

In yet another embodiment, the Conjugate Compound can be delivered in a controlled-release system. In one embodiment, a pump can be used *(see* Langer, *supra;* Sefton, CRC Crit. Ref. Biomed. Eng. 14:201 (1987); Buchwald et al., Surgery 88:507 (1980); Saudek et al., N. Engl. J. Med. 321:574 (1989)). In another embodiment, polymeric materials can be used *(see* Medical Applications of Controlled Release, Langer and Wise (eds.), CRC Pres., Boca Raton, Florida (1974); Controlled Drug Bioavailability, Drug Product Design and Performance, Smolen and Ball (eds.), Wiley, New York (1984); Ranger and Peppas, J. Macromol. Sci. Rev. Macromol. Chem. 23:61 (1983); *see also* Levy et al., Science 228:190 (1985); During et al., Ann. Neurol. 25:351 (1989); Howard et al., J. Neurosurg. 71:105 (1989)). In yet another embodiment, a controlled-release system can be placed in proximity of the target of the Conjugate Compounds, thus requiring only a fraction of the systemic dose *(see, e.g.,* Goodson, in Medical Applications of Controlled Release, supra, vol. 2, pp. 115-138 (1984)). Other controlled-release systems discussed in the review by Langer (Science 249:1527-1533 (1990)) can be used.

In certain embodiments, the present compositions comprise an effective amount of a Conjugate Compound, which can be in isolated and purified form, together with a suitable amount of a pharmaceutically acceptable carrier so as to provide a useful form for administration to the patient.

In a specific embodiment, the term "pharmaceutically acceptable" means approved or approvable by a regulatory agency of the Federal or a state government or listed in the U.S. Pharmacopeia or other generally recognized pharmacopeia for use in animals, and more particularly in humans. The term "carrier" refers to a diluent, adjuvant, excipient, or vehicle with which a Conjugate Compound is administered. Such pharmaceutical carriers can be liquids, such as water and oils, including those of petroleum, animal, vegetable or synthetic origin, such as peanut oil, soybean oil, mineral oil, sesame oil, polymethylated castor oil (CREMAPHOR EL) and the like. The pharmaceutical carriers can be saline, gum acacia, gelatin, starch paste, talc, keratin, colloidal silica, urea, and the like. In addition, auxiliary, stabilizing, thickening, lubricating and coloring agents can be used. When administered to a patient, the Conjugate Compound and pharmaceutically acceptable carriers can be sterile. Water is a useful carrier when the Conjugate Compound is administered intravenously. Saline solutions and aqueous dextrose and glycerol solutions can also be employed as liquid carriers, particularly for injectable solutions. Suitable pharmaceutical carriers also include excipients such as starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The present compositions, if desired, can also contain minor amounts of wetting or emulsifying agents, or pH buffering agents.

The present compositions can take the form of solutions, suspensions, emulsion, tablets, pills, pellets, capsules, capsules containing liquids, powders, sustained-release formulations, suppositories, emulsions, aerosols, sprays, suspensions, or any other form suitable for use. In one embodiment, the pharmaceutically acceptable carrier is a capsule (see e.g., U.S. Patent No. 5,698,155). Other examples of suitable pharmaceutical carriers are described in "Remington's Pharmaceutical Sciences" by E.W. Martin.

In one embodiment, the Conjugate Compounds are formulated in accordance with routine procedures as a pharmaceutical composition adapted for intravenous administration to human beings. Typically, Conjugate Compounds intended for intravenous administration are solutions in sterile isotonic aqueous buffer. Where necessary, the compositions can also include a solubilizing agent. Compositions for intravenous administration can optionally include a local anesthetic such as lignocaine to ease pain at the site of the injection. Generally, the ingredients are supplied either separately or mixed together in unit dosage form, for example, as a dry lyophilized powder or water-free concentrate in a hermetically sealed container such as an ampoule or sachette indicating the quantity of active agent. Where the Conjugate Compound is to be administered by infusion, it can be dispensed, for example, with an infusion bottle containing sterile pharmaceutical grade water or saline. Where the Conjugate Compound is administered by injection, an ampoule of sterile water for injection or saline can be provided so that the ingredients can be mixed prior to administration.

Compositions for oral delivery can be in the form of tablets, lozenges, aqueous or oily suspensions, granules, powders, emulsions, capsules, syrups, or elixirs, for example. Orally administered compositions can contain one or more optionally agents, for example, sweetening agents such as fructose, aspartame or saccharin; flavoring agents such as peppermint, oil of wintergreen, or cherry; coloring agents; and preserving agents, to provide a pharmaceutically palatable preparation. Moreover, where in tablet or pill form, the compositions can be coated to delay disintegration and absorption in the gastrointestinal tract thereby providing a sustained action over an extended period of time. Selectively permeable membranes surrounding an osmotically active driving compound are also suitable for orally administered Conjugate Compound. In these later platforms, fluid from the environment surrounding the capsule is imbibed by the driving compound, which swells to displace the agent or agent composition through an aperture. These delivery platforms can provide an essentially zero order delivery profile as opposed to the spiked profiles of immediate release formulations. A time delay material such as glycerol monostearate or glycerol stearate can also be used. Oral compositions can include standard carriers such as mannitol, lactose, starch, magnesium stearate, sodium saccharine, cellulose, or magnesium carbonate. Such carriers can be of pharmaceutical grade.

The effective amount of the Conjugate Compound depends on the nature of the disorder or condition, and can be determined by standard clinical techniques. In addition, *in vitro or in vivo* assays can optionally be employed to help identify optimal dosage ranges. The precise dose to be employed in the compositions will also depend on the route of administration, and the seriousness of the disease or disorder, and should be decided according to the judgment of the practitioner and each patient's circumstances. However, suitable effective amounts for intravenous administration generally range from about 100 micrograms to about 1 gram per kilogram body weight, in one embodiment from about 200 micrograms to about 500 micrograms, about 400 micrograms to about 2 milligrams, about 1 milligram to about 5 milligram, about 2 milligram to about 20 milligram, about 10 milligram to about 60 milligram, about 50 milligram to about 200 milligram, about 100 milligram to about 500 milligram, or about 200 milligram to about 800 milligram of Conjugate Compound per kilogram body weight. In specific embodiments of the invention, the effective amount ranges from about 0.1 to 1, 0.1 to 20, 0.5 to 15, 0.5 to 5, 1 to 10, 10 to about 40, about 40 to about 60, about 60 to about 100, or about 100 to about 200 milligrams per kilogram body weight.

The invention also provides pharmaceutical packs or kits comprising one or more containers containing one or more Conjugate Compounds. Optionally associated with such container(s) can be instructions for use of one or more Conjugate Compounds or a notice in the form prescribed by a governmental agency regulating the manufacture, use or sale of pharmaceuticals or biological products, which notice reflects approval by the agency of manufacture, use or sale for human administration.

The Conjugate Compounds can be *assayed in vitro,* and then *in vivo,* for the desired activity, prior to use in humans. For *example, in vitro* assays can be used to determine whether administration of a specific Conjugate Compound or combination of Conjugate Compounds is preferred.

In a preferred embodiment, an effective amount of a Conjugate Compound is combined with an excipient and formulated in such a manner that after oral delivery of such a formulation yields adequate concentrations of the Conjugate Compound within the CNS in order to achieve the desired pharmacologic effects *in vivo.* Effective amounts can be determined by routine assays in model systems as described in the section entitled "Assays for use with the Conjugate Compounds" and as illustrated in the Examples.

### Assays for use with the Conjugate Compounds

Several different assays can be employed to test the suitability of a Conjugate Compound. First, the suitability of a Conjugate Compound as a substrate for Aminopeptidase A can be tested by any method known to the skilled artisan. Without being bound by theory, the peptide bond that links the NMDA channel blocker moiety with the Glutamate moiety of a Conjugate Compound is hydrolyzed by Aminopeptidase A resulting in the NMDA receptor antagonist and Glutamate or Glutamate analog. Tests for the inhibition of the NMDA receptor, such as competitive binding with MK-801 and electrophysiological assays using oocytes expressing the NMDA receptor, are well known to the skilled artisan.

The cognition-enhancing effects of a Conjugate Compound can be tested by any assay known to the skilled artisan. Illustrative assays include: acquisition of active avoidance behaviour, lever-holding operant behaviour, use of the radial maze, reversal of scopolamine-induced memory impairment, and reversal of electric shock-induced memory impairment. The neuro-protective effect of a Conjugate Compound can be tested by measuring prevention of NMDA-induced excitotoxicity or prevention of ischemia-induced neuronal cell death.

The following examples exemplify non-limiting aspects of the present invention.

### EXAMPLES

As a prototypic example, the NMDA channel blocker 4,4-dimethyl-cyclohexylamine is linked to glutamate via a peptide bond, to yield Compound 1:

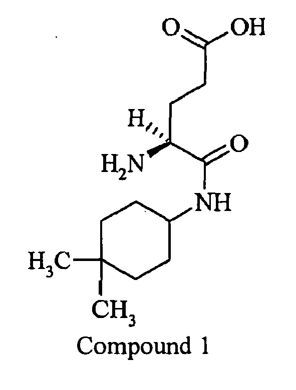

Without being bound by theory, in such a composition, the two active components within Compound 1, the NMDA channel blocker 4,4-dimethyl-cyclohexylamine (henceforth designated Compound 2) and glutamate, can be released upon contact with the endogenous brain endothelial enzyme aminopeptidase A within the CNS.

### Example I: Compound 1 Efficiently Crosses the Blood-Brain Barrier in Rats

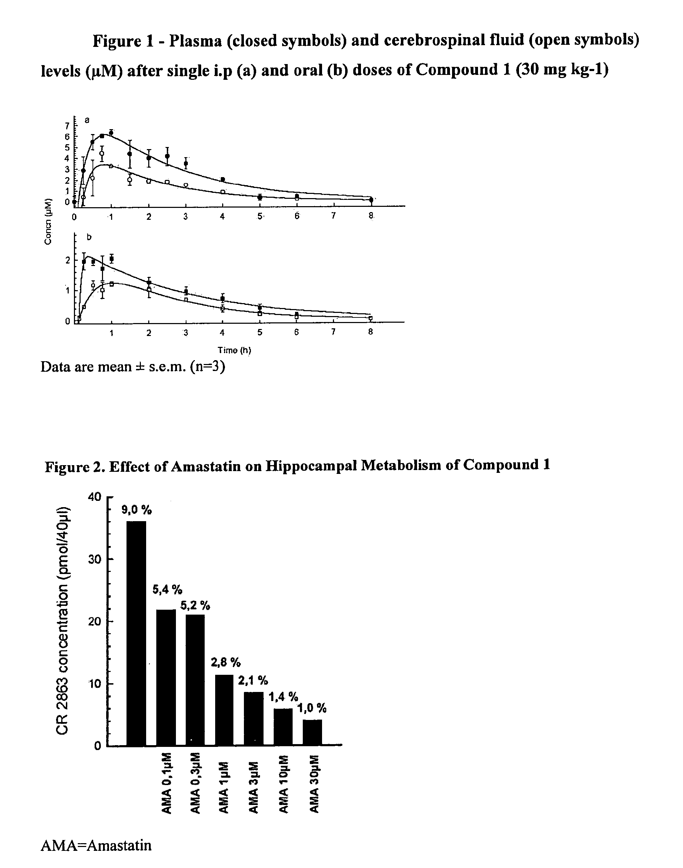

The average (± s.e.m.) plasma and cerebrospinal fluid levels of Compound 1, after a single i.p. or oral administration of 30 mg/kg plotted against time (h), are given in Figure 1. Compound 1 appeared in plasma shortly after both oral and i.p. administration, with a lag-time of 0.12 h. The plasma Tmax was recorded 60 min after both oral and i.p. administration, although plasma levels of Compound 1 given orally were already close to plateau 15 min after administration. Compound 1 crossed the blood-CSF barrier very rapidly, showing lag times of 0.1-0.23 h after oral and i.p. administration, respectively. The maximum concentration of Compound 1 in the CSF was reached 45-60 min after oral and i.p. administration. The CSF/plasma AUC ratios were 48% after i.p. administration and 62% after oral administration. Compound 1 therefore efficiently crosses into the CSF in rats.

### Example II: Compound 1 is Metabolized to Compound 2 plus Glutamate by Aminoptidase A in vitro.

### Materials and Methods

Animals were housed in plastic cages, with free access to standard laboratory food and water, under controlled conditions of lighting, humidity and temperature. Rats were killed by decapitation and brains were quickly removed and immediately transferred in ice-cold artificial cerebrospinal fluid (aCSF) having the following composition (mM):125 NaCl, 3 KCl, 1.2 CaCl₂, 1.2 MgSO₄, 1 NaH₂PO₄, 22 NaHCO₃ and 10 glucose (aerated with 95% O₂ and 5% CO₂ at 37°C), pH 7.2-7.4. Hippocampi were removed on ice and coronal slices chopped (0.40 mm thick). Slices were then incubated for 1 h in the presence of a 95% O₂and 5% CO₂ current (37°C). After washing with fresh aCSF (37°C), slices were transferred to a modified 24 chamber's cell culture well (2 slices per chamber) adapted to accept a constant current of 95% O₂ and 5% CO₂ all experiment time long. 2 ml aCSF was present in each chamber. Some experiments were performed in a modified aCSF containing 1mM EGTA in the absence of Ca²⁺ ions. Drugs were added immediately before slices. After 1h incubation at 37°C, 80 µl aCSF per chamber was drawn in order to evaluate the metabolization time-course. After 3h incubation the experiment ended with a new 80 µl drawing.

### Results

Rat hippocampal slices, incubated in physiological conditions (95% 02; 5% CO2 at 37°C, pH 7.2-7.4), metabolized Compound 1 ((S)-4-amino-5-[(4,4-dimethylcyclohexyl)-amino]-5-oxo-pentanoic acid) into Compound 2 (4,4-dimethyl-cyclohexamine) + glutamic acid. After 1h incubation 2,5% ± 0,3% (n=2) Compound 1 was cleaved to yield Compound 2. After 3h incubation 9,0% ± 0,0% (n=2) Compound 1 was cleaved to Compound 2. In the presence of 3 µM amastatin, a selective inhibitor of aminopeptidase A, the cleavage of Compound 1 at 1h and 3h was reduced to 0,5% and 2,0%, respectively (see Figure 2). Amastatin dose-dependently inhibited cleavage of Compound 1 with an IC50 of 0,3µM. Since aminopeptidase A is a Ca++-dependent enzyme, the cleavage of Compound 1 was then examined under Ca++ -free conditions. In the absence of Ca++ ions, Compound 1 cleavage was inhibited by 76% at 1h and 3h. Aminopeptidase A therefore appears to be the principal enzyme responsible for the cleavage of Compound 1 into Compound 2 plus glutamate.

### Example III: Compound 2 inhibits the binding of the open-channel ligand MK801 to the NMDA receptor

MK-801 (dizocilpine) is an open-channel ligand of the NMDA Ca channel. Washed rat cortical membranes enriched for NMDA receptors were incubated with increasing concentrations of the co-agonists glycine or glutamate, or the positive modulator spermine, and Compound 2. The binding of ³H MK801 was then measured. Compound 2 inhibited MK-801 binding in a dose-dependent fashion (see Figure 3A). Compound 2 inhibited enhancement of MK-801 binding by the co-agonists glycine and glutamate, as well as the positive modulator spermine. These results strongly suggest that Compound 2 binds to the NMDA Ca channel at the same site as MK-801.

### Example IV: Compound 2 is a noncompetitive, voltage-dependent NMDA channel blocker

Glutamate stimulates an inward Ca current through the NMDA receptor channel by binding to a specific ligand-binding site at the NR1/NR2 subunit interface and opening the ion channel to the influx of calcium. Glutamate (10uM) was added to oocytes transfected with the NMDAR (subtype NR1/NR2A) in the presence of 100uM glycine and increasing amounts of Compound 2. The noncompetitive, voltage-dependent channel blocker memantine was used as a positive control. Compound 2 added either prior to or concurrent with the addition of glutamate inhibited the glutamate-stimulated Ca current, with an IC₅₀ of 117 and 30uM at -60 and 100 millivolts, respectively (see Figure 3B). Compound 2 acted similarly to memantime, though memantine was approx. 100-times more potent than Compound 2, having an IC₅₀ of 0.75 and 0.23uM at -60 and 100 millivolts respectively (results not shown). These results demonstrate that Compound 2 is a reversible, voltage-dependent NMDA open channel blocker, similar to, but less potent than the known voltage-dependent open channel-blocker memantine.

### Example V: Compound 2, but not Compound 1, protects against NMDA excito-toxicity in vitro

In this experiment, slices were obtained from hippocampi of young (8 day old) rats. After one hour of pre-incubation, NMDA was added to give a final concentration of 100 µM. Either Compound 1 or Compound 2 was added 15 min prior to NMDA and the incubation was carried out for 45 min. Three slices of each sample were collected and fixed with glutaraldehyde (5%). After embedding and freezing, sections (4 µm) were stained with Cresyl violet. Neuronal injury was studied with light microscopy using an imaging analyser (Kontron, KS300). The area occupied by CA1 pyramidal neurons was measured in an analyser-defined field. The pattern of hippocampal neuro-degeneration following NMDA-stimulation consisted of a significant decrease in neuronal nuclei area. Compound 2 dose-dependently (IC₅₀ = 188.8 ± 42.4 µM) prevented NMDA-induced damage (see Figure 4). In contrast, Compound 1 had no effect on NMDA-induced neuronal damage (results not shown).

### Example VI: Compound 2 is neuro-protective in vivo

This experiment investigated the neuro-protective effects of Compound 2 in gerbils subjected to bilateral occlusion of the carotid arteries. Mongolian gerbils were anaesthetised with halothane and the right and left common carotid arteries were occluded for a period of 5 min. The animals were observed for 7 days, and surviving animals were then sacrificed. The brains were cut into 10 µm-thick sections in the area of the hippocampus and sections were stained with cresyl violet. Quantitative assessment of area occupied by CA pyramidal neurons was performed using an image analyser (Kontron, KS300). For each animal, measurements were made in a defined field from both hippocampi in 4 coronal sections. The raw data were represented by the mean of 8 measures. The pattern of hippocampal neuro-degeneration following ischemia consisted of a significant decrease of neuronal nuclei area.

When administered 1 hour prior to ischemia, Compound 2 dose-dependently protected animals from neuronal degeneration and death. Ischemia resulted in a 7-day mortality of 50%. Compound 2 at a dose of 16 mg/kg, was 100% protective (see Figure 5). Compound 1 was less potent, reducing mortality to zero at a dose of 50mg/kg. These results indicate that the neuroprotective effects of Compound 1 are mediated by the NMDAR channel blocker Compound 2.

### Example VIII: Compound 1, but not Compound 2, enhances learning in normal animals

The acquisition of active avoidance behaviour was evaluated during one 40-trial daily session for 5 consecutive days. Compound 1, when given at doses of 3 and 10 mg/kg, increased the percent of conditioned avoidance (see Figure 6A). The regression slopes using one way analysis of variance (ANOVA) were compared, and revealed a significant treatment effect (p<0.001). Post-hoc comparison (Dunnett's method) showed that animals treated with Compound 1 at doses of 3 and 10 mg/kg had a faster improvement, suggesting a faster learning. Compound 1 did not affect the uncoordinated escape responses or spontaneous inter-trial responses. Compound 2 was without activity in this model (results not shown), strongly suggesting that the learning-enhancing effect was due to the release of glutamate from Compound 1.

In a further test of learning, lever-holding operant behaviour was tested in a standard rodent operant conditioning chamber (Skinner Box). Rats were trained to press the lever for water reward on a continuous reinforcement schedule, for which each lever pressing was rewarded. Daily sessions lasted 30 min and were performed under a green light. Water was restricted to a 30 min post-session period. After two days of stabilisation, rats were subjected to a visual discrimination schedule, in which a green light signalled the availability of the reinforcement for 60 sec, followed by a 30 sec interval with no reinforcement. Daily sessions lasted 30 min and were repeated for 14 days. Data were expressed as the Efficiency Index, which is a ratio of reinforced (correct responses during a session) to non-reinforced responses.

Compound 1 at doses of 3-10-30 mg/kg i.p. increased in a dose-dependent manner the percent of reinforced responses (Efficiency Index). ANOVA split-plot analysis showed a significant effect of treated groups versus control [F (3,36) = 4.76 p=0.007], and a significant effect across time (p<0.001), demonstrating that the dose-effect regression increased over the time (see Figure 6B).

In another experiment of learning, a radial maze test was used. The radial maze consisted of eight arms radiating from a centre platform. The platform was 40 cm in diameter and the eight arms were 60 cm long, 13 cm wide, with walls 10 cm high. The surface of the maze was elevated 50 cm from the floor. Plastic food cups (4 cm in diameter) were at the end of each arm. One 50 mg micro-pellet was placed in a cup at the beginning of each trial. In the training session, the rat was placed in the centre of the platform facing arm #4 and allowed to freely explore the maze. The animal was left in the maze until it ate all of the 24 pellets placed three per arm.

Rats treated with Compound 1 (1-30 mg/kg) had faster test acquisition in the radial maze than control animals. Pair-wise comparison between treatment groups showed that Compound 1 at doses of 10 and 30 mg/kg decreased the number of sessions to reach acquisition criterion (P <0.05, P <0.01, respectively) (see Figure 6C). In contrast, Compound 2 was without effect, demonstrating that the learning-enhancing effect was due to the glutamate released from Compound 1.

### Example IX: Compound 1 reverses induced memory impairment in animals

In this experiment, Compound 1 was tested to determine whether it could reverse scopolamine-induced memory impairment in a passive-avoidance paradigm in rats. Scopolamine-treated rats had a significantly shorter retention-latency than saline-treated control rats. Compound 1 (1 and 10 mg/kg, i.p.) dose-dependently increased latency; reaching significance (p<0.05) at the 10 mg/kg dose(see Figure 7A). In contrast, Compound 2 was unable to reverse the amnesic effect of scopolamine (results not shown), demonstrating that the amnesic-reversing effect of Compound 1 was due to the released glutamate.

In another model of memory impairment, electroconvulsive shock was employed as a memory de-stabilizer. In this experiment, electroconvulsive shock resulted in significantly lower retention latency (P <0.01) compared to non-shocked controls. Compound 1 at doses of 3, 10 and 30 mg/kg dose-dependently increased step-down latency. For doses of 10 and 30 mg/kg the effect was statistically significant (P <0.05 and P <0.01, respectively) (see Figure 7B). Again, Compound 2 was without effect in this mode (results not shown).

The present invention is not to be limited in scope by the specific embodiments disclosed in the examples which are intended as illustrations of a few aspects of the invention and any embodiments that are functionally equivalent are within the scope of this invention. Indeed, various modifications of the invention in addition to those shown and described herein will become apparent to those skilled in the art and are intended to fall within the scope of the appended claims.

A number of references have been cited, the entire disclosures of which have been incorporated herein in their entirety.

## Claims

1. A compound having the Formula (I): 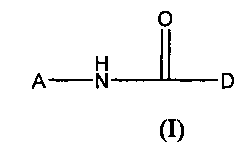 or a pharmaceutically acceptable salt or solvate thereof, wherein:
A is an NMDA channel blocker; and
D is: 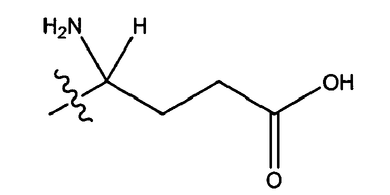 with the proviso that the compound is not:

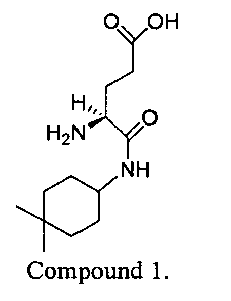

2. The compound of claim 1, wherein A is selected from the group consisting of: D(-)-2-amino-4-phosphonobutyric acid, D(-)-2-amino-7-phosphonoheptanoic acid, D(-)-2-amino-5-phosphonopentanoic acid, DL-2-amino-5-phosphonopentanoic acid, R(-)-3-(2-carboxypiperazin-4-yl)-propyl-1-phosphonic acid, (RS)-3-(2-carboxypiperazin-4-yl)-propyl-1-phosphonic acid, 4-Cl-kynurenine, 7-chloro-kynurenic acid, (-)6-phosphonomethyl-decahydroi- soquinoline-3-carboxylic acid, ACPC, aptiganel, besonprodil, BMY-14802, budipine, CGP-37849, CP-101606, conantokin G, CR-3991, CR-2249, CR-3394, delucemine, dexanabinol, dizocilpine, EAA-090, eliprodil, felbamate, fluorofelbamate, FPL-12495, gacyclidine, gavestinel, glycine, harkoseride, HU-211, ibogaine, ipenoxazone, kaitocephalin, ketamine, L-695902, lanicemine, licostinel, ligustizine, midafotel, milnacipran, neboglamine, nebostinel, neramexane, N'-[2-chloro-5-(methylthio)phenyl]-N- -methyl-N-[3-(methylthio)phenyl]-guanidine, N'-[2-chloro-5-(methylthio)phenyl]-N-methyl-N-[3-[(R)-methylsulfinyl]phenyl]-guanidine, neramexane, orphenadrine, remacemide, RGH-896, RG-13484, RG-13579, RG-1103, Ro-25-6981, selfotel, seratrodast, spermidine, spermine, topiramate, traxoprodil, UK-240255, ZD-9379, .alpha.-amino-2-(2-phosphonoethyl)-cyclo- hexanepropanoic acid, ,alpha.-amino-4-(phosphonomethyl)-benzeneacetic acid, N(1)-(benzyl)cinnamamidine, and 4-benzyl-4-hydroxy-N-(hydroxyphenox- yalkyl)-piperidine, Adamantane, 5-(2-amino-2-carboxyethyl)-4,5-dihydroisoxazole-3-carboxylic acid, Memantine, Cyclohexylbenzene, N-[2-(3,5-dimethyl-1-adamantyl)ethyl] guanidine, N-[2-(3,5-dimethyl-1-adamantyl) ethyl]acetamidine, (+)-SKF38393, and Dopamine.

3. A compound having the formula: 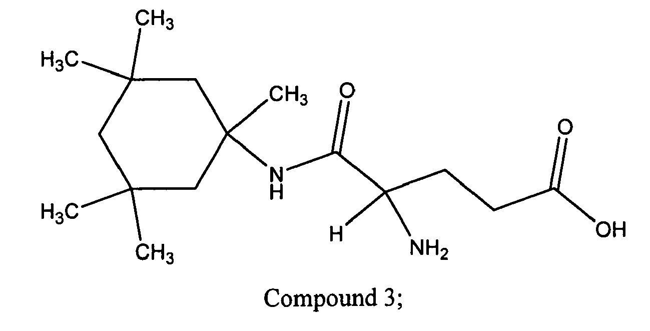 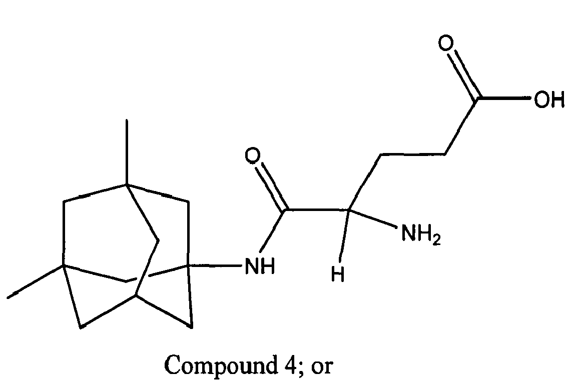 a pharmaceutically acceptable salt or solvate thereof.

4. The compound of claim 3, wherein the compound is: 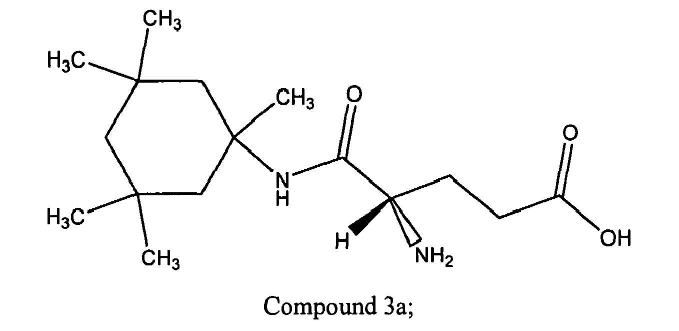 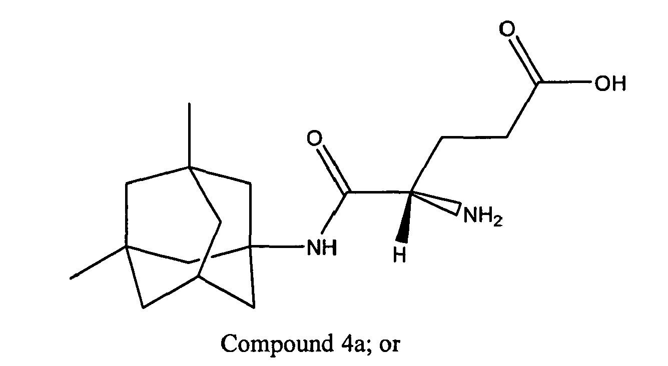 a pharmaceutically acceptable salt or solvate thereof.

5. A pharmaceutical composition comprising (i) a compound having the Formula (I): 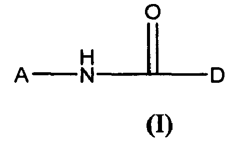 or a pharmaceutically acceptable salt or solvate thereof, wherein:
A is an NMDA channel blocker; and
D is: 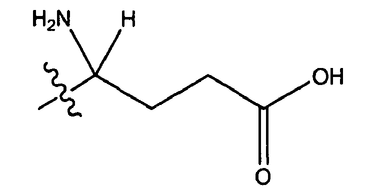 with the proviso that the compound is not: 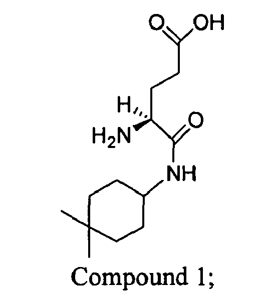 and (ii) a pharmaceutically acceptable carrier.

6. The pharmaceutical composition of claim 5, wherein A is selected from the group consisting of:
D(-)-2-amino-4-phosphonobutyric acid, D(-)-2-amino-7-phosphonoheptanoic acid, D(-)-2-amino-5-phosphonopentanoic acid, DL-2-amino-5-phosphonopentanoic acid, R(-)-3-(2-carboxypiperazin-4-yl)-propyl-1-phosphonic acid, (RS)-3-(2-carboxypiperazin-4-yl)-propyl-1-phosphonic acid, 4-Cl-kynurenine, 7-chloro-kynurenic acid, (-)6-phosphonomethyl-decahydroi- soquinoline-3-carboxylic acid, ACPC, aptiganel, besonprodil, BMY-14802, budipine, CGP-37849, CP-101606, conantokin G, CR-3991, CR-2249, CR-3394, delucemine, dexanabinol, dizocilpine, EAA-090, eliprodil, felbamate, fluorofelbamate, FPL-12495, gacyclidine, gavestinel, glycine, harkoseride, HU-211, ibogaine, ipenoxazone, kaitocephalin, ketamine, L-695902, lanicemine, licostinel, ligustizine, midafotel, milnacipran, neboglamine, nebostinel, neramexane, N'-[2-chloro-5-(methylthio)phenyl]-N- -methyl-N-[3-(methylthio)phenyl]-guanidine, N'-[2-chloro-5-(methylthio)phenyl]-N-methyl-N-[3-[(R)-methylsulfinyl]phenyl]-guanidine, neramexane, orphenadrine, remacemide, RGH-896, RG-13484, RG-13579, RG-1103, Ro-25-6981, selfotel, seratrodast, spermidine, spermine, topiramate, traxoprodil, UK-240255, ZD-9379, .alpha.-amino-2-(2-phosphonoethyl)-cyclo- hexanepropanoic acid, .alpha.-amino-4-(phosphonomethyl)-benzeneacetic acid, N(1)-(benzyl)cinnamamidine, and 4-benzyl-4-hydroxy-N-(hydroxyphenox- yalkyl)-piperidine, Adamantane, 5-(2-amino-2-carboxyethyl)-4,5-dihydroisoxazole-3-carboxylic acid, Memantine, Cyclohexylbenzene, N-[2-(3,5-dimethyl-l-adamantyl)ethyl] guanidine, N-[2-(3,5-dimethyl-1-adamantyl) ethyl]acetamidine, (+)-SKF38393, and Dopamine.

7. A pharmaceutical composition comprising (i) a compound having the formula: 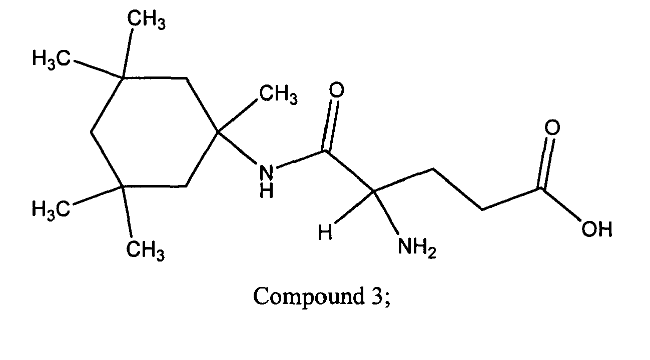 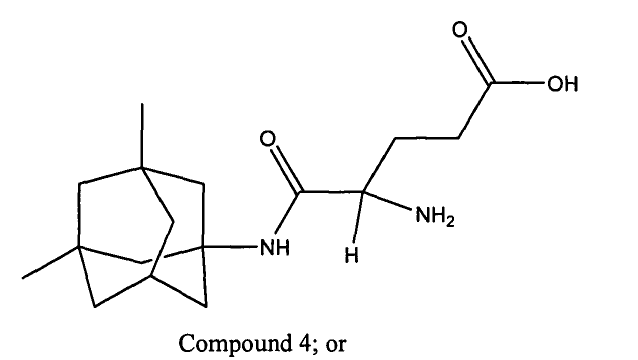 a pharmaceutically acceptable salt or solvate thereof; and (ii) a pharmaceutically acceptable carrier.

8. The pharmaceutical composition of claim 7, wherein the compound is: 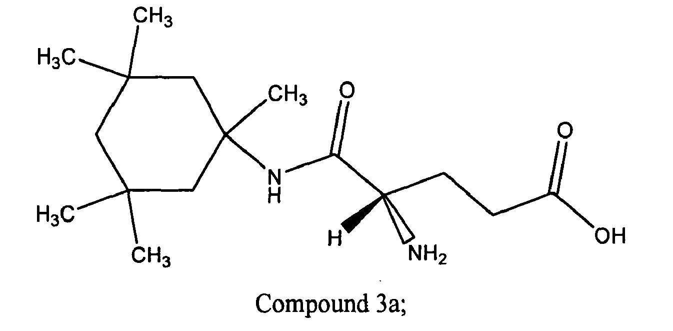 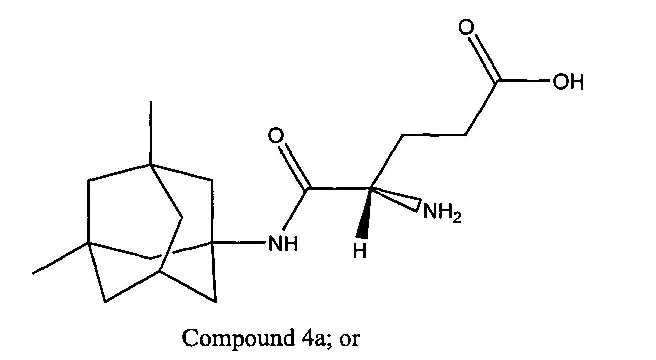 a pharmaceutically acceptable salt or solvate thereof.

9. A method for treating or preventing a neurodegenerative disorder in a subject,
wherein said method comprises administering to the subject an effective amount of a compound having the Formula (I): 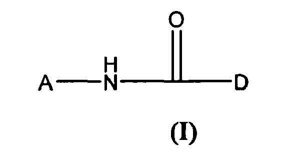 or a pharmaceutically acceptable salt or solvate thereof, wherein:
A is an NMDA channel blocker; and
D is: 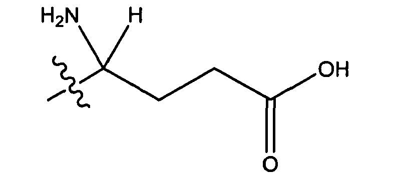 with the proviso that the compound is not:

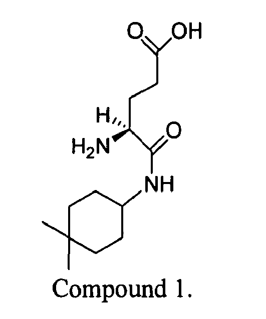

10. The method of claim 9, wherein A is selected from the group consisting of: D(-)-2-amino-4-phosphonobutyric acid, D(-)-2-amino-7-phosphonoheptanoic acid, D(-)-2-amino-5-phosphonopentanoic acid, DL-2-amino-5-phosphonopentanoic acid, R(-)-3-(2-carboxypiperazin-4-yl)-propyl-1-phosphonic acid, (RS)-3-(2-carboxypiperazin-4-yl)-propyl-1-phosphonic acid, 4-Cl-kynurenine, 7-chloro-kynurenic acid, (-)6-phosphonomethyl-decahydroi- soquinoline-3-carboxylic acid, ACPC, aptiganel, besonprodil, BMY-14802, budipine, CGP-37849, CP-101606, conantokin G, CR-3991, CR-2249, CR-3394, delucemine, dexanabinol, dizocilpine, EAA-090, eliprodil, felbamate, fluorofelbamate, FPL-12495, gacyclidine, gavestinel, glycine, harkoseride, HU-211, ibogaine, ipenoxazone, kaitocephalin, ketamine, L-695902, lanicemine, licostinel, ligustizine, midafotel, milnacipran, neboglamine, nebostinel, neramexane, N'-[2-chloro-5-(methylthio)phenyl]-N- -methyl-N-[3-(methylthio)phenyl]-guanidine, N'-[2-chloro-5-(methylthio)phenyl]-N-methyl-N-[3-[(R)-methylsulfinyl]phenyl]-guanidine, neramexane, orphenadrine, remacemide, RGH-896, RG-13484, RG-13579, RG-1103, Ro-25-6981, selfotel, seratrodast, spermidine, spermine, topiramate, traxoprodil, UK-240255, ZD-9379, .alpha.-amino-2-(2-phosphonoethyl)-cyclo- hexanepropanoic acid, alpha. -amino-4-(phosphonomethyl)-benzeneacetic acid, N(1)-(benzyl)cinnamamidine, and 4-benzyl-4-hydroxy-N-(hydroxyphenok- yalkyl)-piperidine, Adamantane, 5-(2-amino-2-carboxyethyl)-4,5-dihydroisoxazole-3-carboxylic acid, Memantine, Cyclohexylbenzene, N-[2-(3,5-dimethyl-1-adamantyl)ethyl] guanidine, N-[2-(3,5-dimethyl-1-adamantyl) ethyl]acetamidine, (+)-SKF38393, and Dopamine.

11. A method for treating or preventing a neurodegenerative disorder in a subject,
wherein said method comprises administering to the subject an effective amount of a compound having the formula: 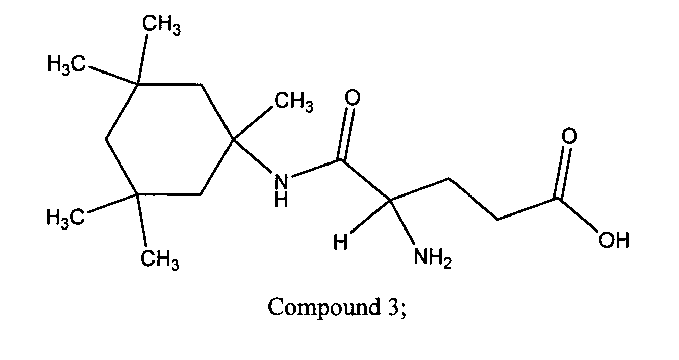 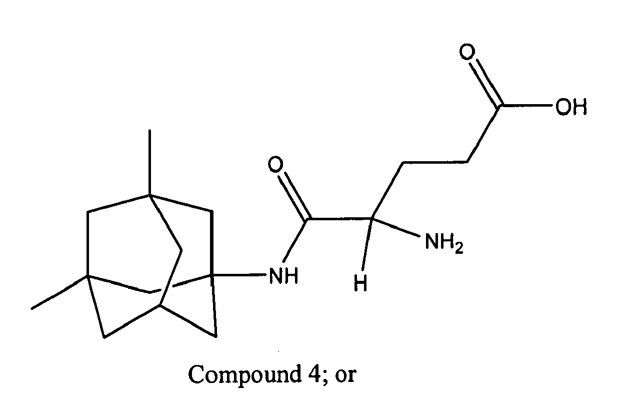 a pharmaceutically acceptable salt or solvate thereof.

12. The method of claim 11 wherein the compound is: 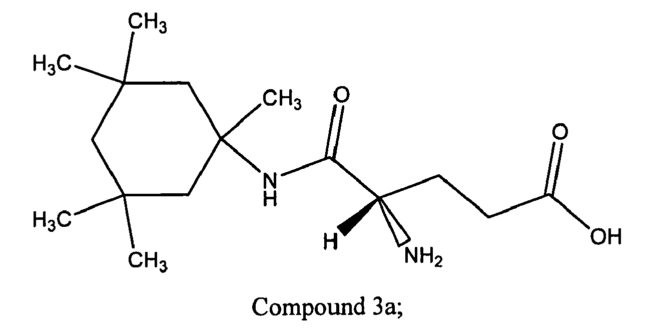 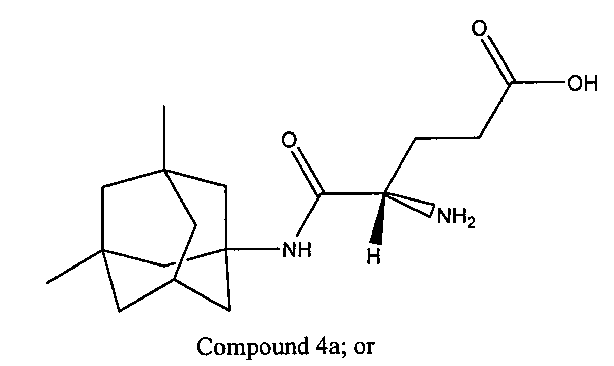 a pharmaceutically acceptable salt or solvate thereof.

13. The method of claim 9, 10, 11, or 12, wherein the neurodegenerative disorder is selected from the group consisting of: Alzheimer's disease, vascular dementia, Parkinson's disease, and traumatic brain injury.

14. A method for enhancing learning, memory, and cognition in a subject, wherein said method comprises administering to the subject an effective amount of a compound having the Formula (I): 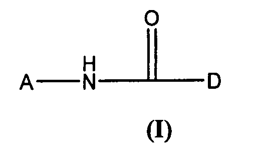 or a pharmaceutically acceptable salt or solvate thereof, wherein:
A is an NMDA channel blocker; and
D is: 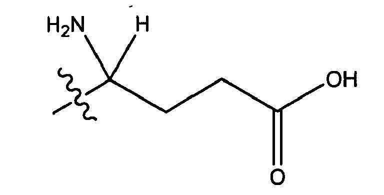 with the proviso that the compound is not:

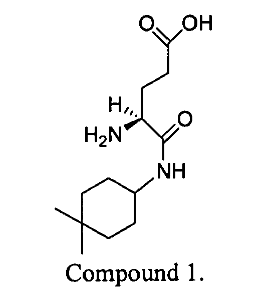

15. The method of claim 149, wherein A is selected from the group consisting of: D(-)-2-amino-4-phosphonobutyric acid, D(-)-2-amino-7-phosphonoheptanoic acid, D(-)-2-amino-5-phosphonopentanoic acid, DL-2-amino-5-phosphonopentanoic acid, R(-)-3-(2-carboxypiperazin-4-yl)-propyl-1-phosphonic acid, (RS)-3-(2-carboxypiperazin-4-yl)-propyl-1-phosphonic acid, 4-Cl-kynurenine, 7-chloro-kynurenic acid, (-)6-phosphonomethyl-decahydroi- soquinoline-3-carboxylic acid, ACPC, aptiganel, besonprodil, BMY-14802, budipine, CGP-37849, CP-101606, conantokin G, CR-3991, CR-2249, CR-3394, delucemine, dexanabinol, dizocilpine, EAA-090, eliprodil, felbamate, fluorofelbamate, FPL-12495, gacyclidine, gavestinel, glycine, harkoseride, HU-211, ibogaine, ipenoxazone, kaitocephalin, ketamine, L-695902, lanicemine, licostinel, ligustizine, midafotel, milnacipran, neboglamine, nebostinel, neramexane, N'-[2-chloro-5-(methylthio)phenyl]-N- -methyl-N-[3-(methylthio)phenyl]-guanidine, N'-[2-chloro-5-(methylthio)phenyl]-N-methyl-N-[3-[(R)-methylsulfinyl]phenyl]-guanidine, neramexane, orphenadrine, remacemide, RGH-896, RG-13484, RG-13579, RG-1103, Ro-25-6981, selfotel, seratrodast, spermidine, spermine, topiramate, traxoprodil, UK-240255, ZD-9379, .alpha.-amino-2-(2-phosphonoethyl)-cyclo- hexanepropanoic acid, .alpha.-amino-4-(phosphonomethyl)-benzeneacetic acid, N(1)-(benzyl)cinnamamidine, and 4-benzyl-4-hydroxy-N-(hydroxyphenox- yalkyl)-piperidine, Adamantane, 5-(2-amino-2-carboxyethyl)-4,5-dihydroisoxazole-3-carboxylic acid, Memantine, Cyclohexylbenzene, N-[2-(3,5-dimethyl-1-adamantyl)ethyl] guanidine, N-[2-(3,5-dimethyl-1-adamantyl) ethyl]acetamidine, (+)-SKF38393, and Dopamine.

16. A method for enhancing learning, memory, and cognition in a subject, wherein said method comprises administering to the subject an effective amount of a compound having the formula: 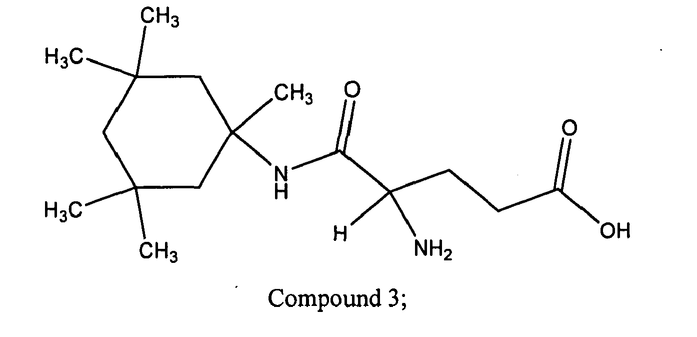 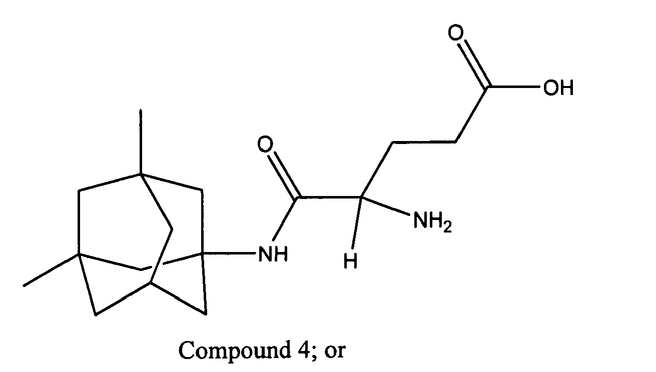 a pharmaceutically acceptable salt or solvate thereof.

17. The method of claim 16 wherein the compound is: 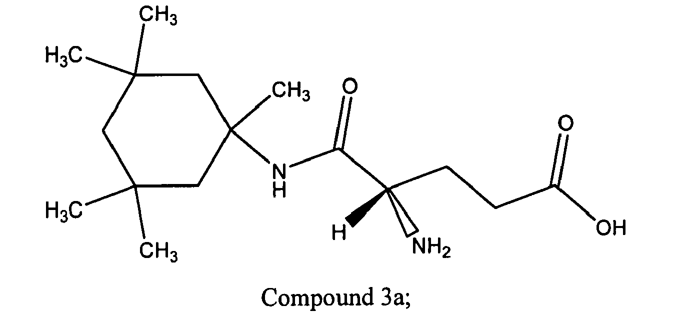 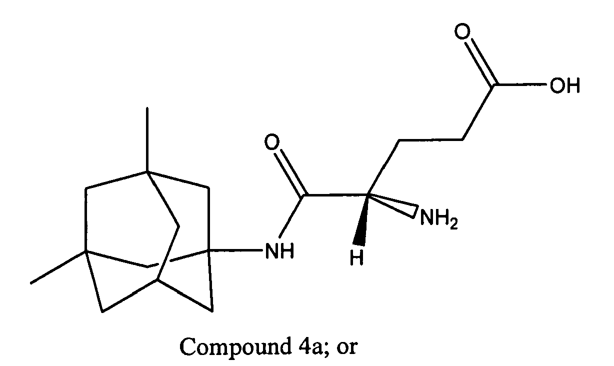 a pharmaceutically acceptable salt or solvate thereof.

18. A method for treating a neurodegenerative disorder in a subject, said method comprising administering to the subject and effective amount of an NMDA channel blocker in combination with an effective amount of glutamate.

19. The method of claim 18, wherein said combination of an NMDA channel blocker and glutamate is administered directly into the brain of the subject.

20. The method of claim 18 or 19, wherein said combination of an NMDA channel blocker and glutamate is administered via an implant.

21. The method of claim 20, wherein the implant supports the slow release of said combination of an NMDA channel blocker and glutamate.

22. The method of claim 9, 10, 11, 12, 14, 15, 16, 17, 18, 19, or 21, wherein the subject is a human.

23. The method of claim 9, 10, 11, 12, 14, 15, 16, 17, 18, 19, or 21, wherein the route of administration is via oral delivery, via systemic delivery, via implantation, or via sustained release.

24. A compound having the Formula (I): 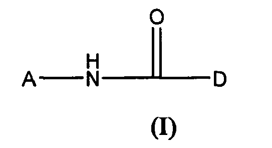 or a pharmaceutically acceptable salt or solvate thereof, wherein:
A is an NMDA channel blocker; and
D is: 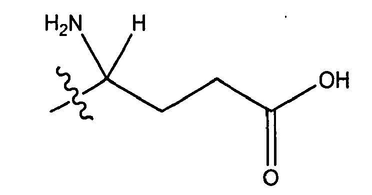 with the proviso that the compound is not: 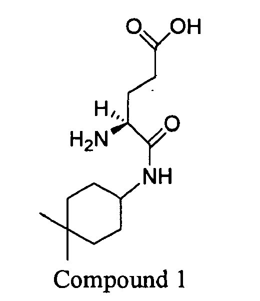 for use as a medicament.

25. The compound of claim 24, wherein A is selected from the group consisting of: D(-)-2-amino-4-phosphonobutyric acid, D(-)-2-amino-7-phosphonoheptanoic acid, D(-)-2-amino-5-phosphonopentanoic acid, DL-2-amino-5-phosphonopentanoic acid, R(-)-3-(2-carboxypiperazin-4-yl)-propyl-1-phosphonic acid, (RS)-3-(2-carboxypiperazin-4-yl)-propyl-1-phosphonic acid, 4-Cl-kynurenine, 7-chloro-kynurenic acid, (-)6-phosphonomethyl-decahydroi- soquinoline-3-carboxylic acid, ACPC, aptiganel, besonprodil, BMY-14802, budipine, CGP-37849, CP-101606, conantokin G, CR-3991, CR-2249, CR-3394, delucemine, dexanabinol, dizocilpine, EAA-090, eliprodil, felbamate, fluorofelbamate, FPL-12495, gacyclidine, gavestinel, glycine, harkoseride, HU-211, ibogaine, ipenoxazone, kaitocephalin, ketamine, L-695902, lanicemine, licostinel, ligustizine, midafotel, milnacipran, neboglamine, nebostinel, neramexane, N'-[2-chloro-5-(methylthio)phenyl]-N- -methyl-N-[3-(methylthio)phenyl]-guanidine, N'-[2-chloro-5-(methylthio)phenyl]-N-methyl-N-[3-[(R)-methylsulfinyl]phenyl]-guanidine, neramexane, orphenadrine, remacemide, RGH-896, RG-13484, RG-13579, RG-1103, Ro-25-6981, selfotel, seratrodast, spermidine, spermine, topiramate, traxoprodil, UK-240255, ZD-9379, alpha. -amino-2-(2-phosphonoethyl)-cyclo- hexanepropanoic acid, .alpha.-amino-4-(phosphonomethyl)-benzeneacetic acid, N(1)-(benzyl)cinnamamidine, and 4-benzyl-4-hydroxy-N-(hydroxyphenox- yalkyl)-piperidine, Adamantane, 5-(2-amino-2-carboxyethyl)-4,5-dihydroisoxazole-3-carboxylic acid, Memantine, Cyclohexylbenzene, N-[2-(3,5-dimethyl-1-adamantyl)ethyl] guanidine, N-[2-(3,5-dimethyl-1-adamantyl) ethyl]acetamidine, (+)-SKF38393, and Dopamine.

26. A compound having the formula: 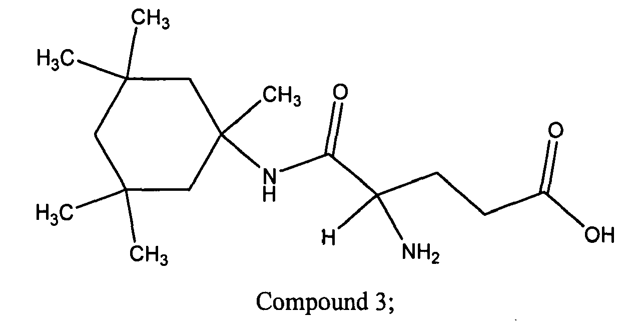 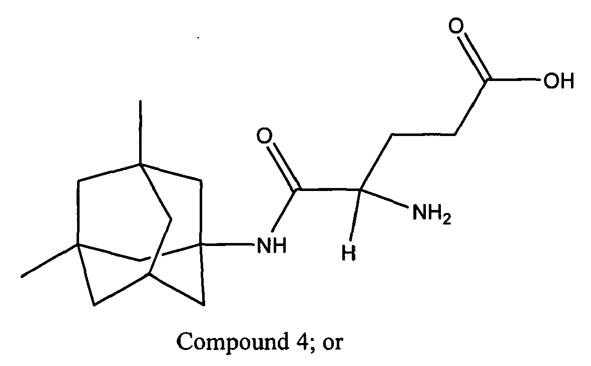 a pharmaceutically acceptable salt or solvate thereof for use as a medicament.

27. The compound of claim 26, wherein the compound is: 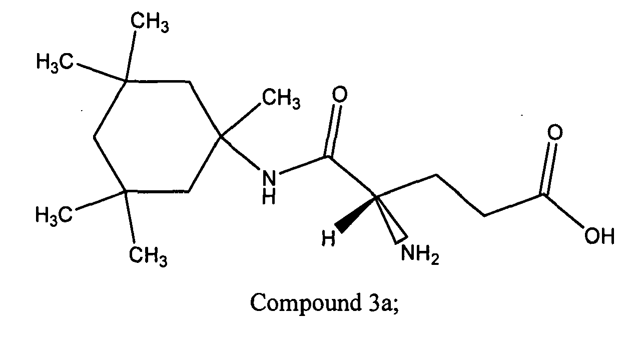 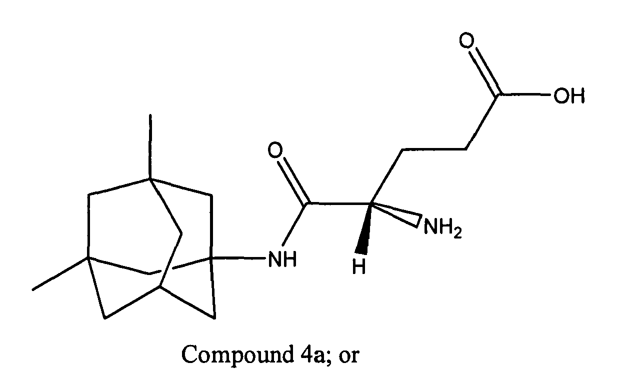 a pharmaceutically acceptable salt or solvate thereof.

28. Use of a compound having the Formula (I): 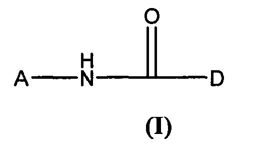 or a pharmaceutically acceptable salt or solvate thereof, wherein:
A is an NMDA channel blocker; and
D is: 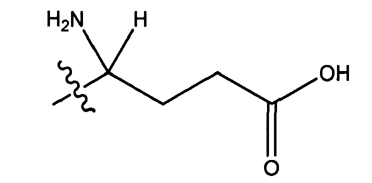 with the proviso that the compound is not: 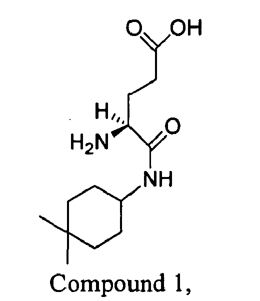 for the manufacture of a medicament for treating or preventing a neurodegenerative disorder.

29. The use of claim 28 wherein A is selected from the group consisting of: D(-)-2-amino-4-phosphonobutyric acid, D(-)-2-amino-7-phosphonoheptanoic acid, D(-)-2-amino-5-phosphonopentanoic acid, DL-2-amino-5-phosphonopentanoic acid, R(-)-3-(2-carboxypiperazin-4-yl)-propyl-1-phosphonic acid, (RS)-3-(2-carboxypiperazin-4-yl)-propyl-1-phosphonic acid, 4-Cl-kynurenine, 7-chloro-kynurenic acid, (-)6-phosphonomethyl-decahydroi- soquinoline-3-carboxylic acid, ACPC, aptiganel, besonprodil, BMY-14802, budipine, CGP-37849, CP-101606, conantokin G, CR-3991, CR-2249, CR-3394, delucemine, dexanabinol, dizocilpine, EAA-090, eliprodil, felbamate, fluorofelbamate, FPL-12495, gacyclidine, gavestinel, glycine, harkoseride, HU-211, ibogaine, ipenoxazone, kaitocephalin, ketamine, L-695902, lanicemine, licostinel, ligustizine, midafotel, milnacipran, neboglamine, nebostinel, neramexane, N'-[2-chloro-5-(methylthio)phenyl]-N- -methyl-N-[3-(methylthio)phenyl]-guanidine, N'-[2-chloro-5-(methylthio)phenyl]-N-methyl-N-[3-[(R)-methylsulfinyl]phenyl]-guanidine, neramexane, orphenadrine, remacemide, RGH-896, RG-13484, RG-13579, RG-1103, Ro-25-6981, selfotel, seratrodast, spermidine, spermine, topiramate, traxoprodil, UK-240255, ZD-9379, alpha. -amino-2-(2-phosphonoethyl)-cyclo- hexanepropanoic acid, alpha. -amino-4-(phosphonomethyl)-benzeneacetic acid, N(1)-(benzyl)cinnamamidine, and 4-benzyl-4-hydroxy-N-(hydroxyphenox- yalkyl)-piperidine, Adamantane, 5-(2-amino-2-carboxyethyl)-4,5-dihydroisoxazole-3-carboxylic acid, Memantine, Cyclohexylbenzene, N-[2-(3,5-dimethyl-1-adamantyl)ethyl] guanidine, N-[2-(3,5-dimethyl-1-adamantyl) ethyl]acetamidine, (+)-SKF38393, and Dopamine.

30. Use of a compound having the formula: 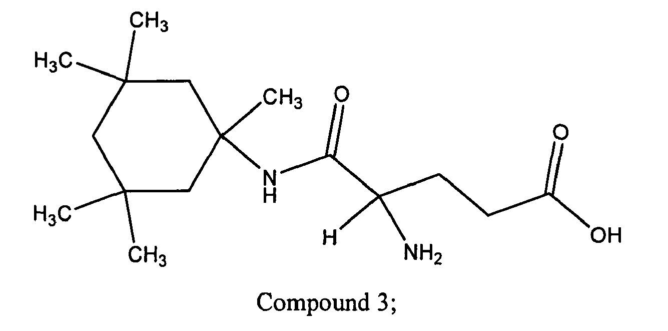 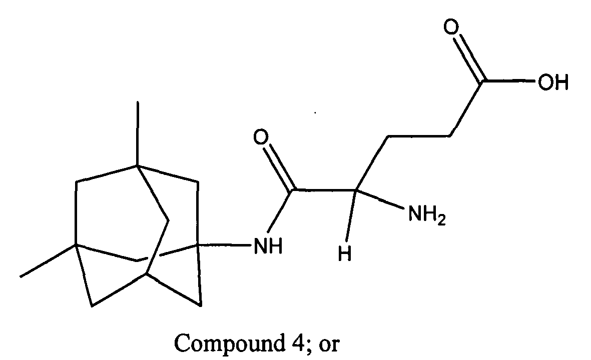 a pharmaceutically acceptable salt or solvate thereof for the manufacture of a medicament to treat or prevent a neurodegenerative disorder.

31. The use of claim 30, wherein the compound is: 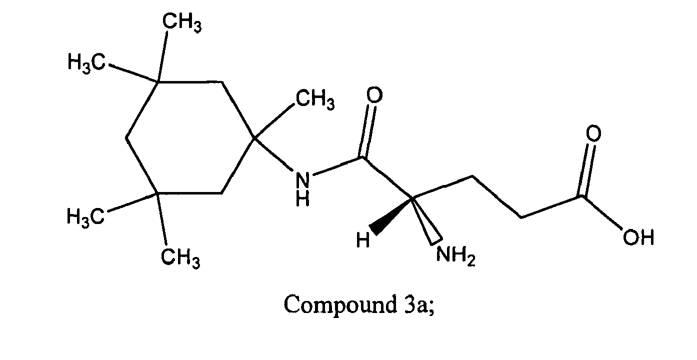 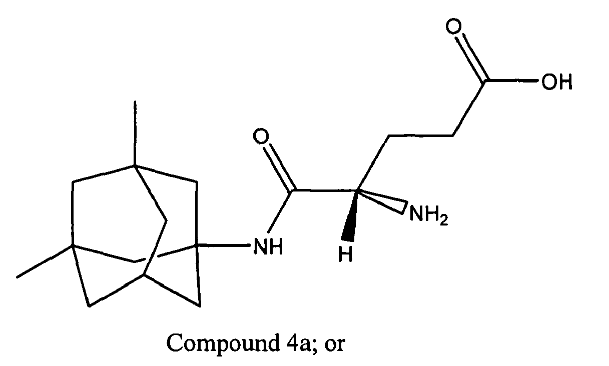 a pharmaceutically acceptable salt or solvate thereof.

32. The use of claim 28, 29, 30, or 31, wherein the neurodegenerative disorder is selected from the group consisting of: Alzheimer's disease, vascular dementia, Parkinson's disease, and traumatic brain injury.

33. Use of a compound having the Formula (I): 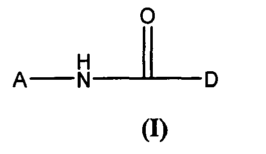 or a pharmaceutically acceptable salt or solvate thereof, wherein:
A is an NMDA channel blocker; and
D is: 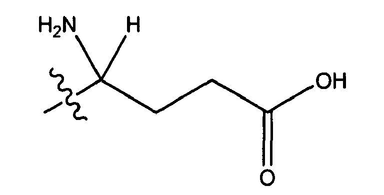 with the proviso that the compound is not: 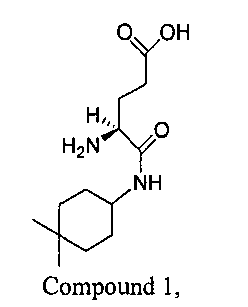 for the manufacture of a medicament for enhancing learning, memory, or cognition.

34. The use of claim 33 wherein A is selected from the group consisting of: D(-)-2-amino-4-phosphonobutyric acid, D(-)-2-amino-7-phosphonoheptanoic acid, D(-)-2-amino-5-phosphonopentanoic acid, DL-2-amino-5-phosphonopentanoic acid, R(-)-3-(2-carboxypiperazin-4-yl)-propyl-1-phosphonic acid, (RS)-3-(2-carboxypiperazin-4-yl)-propyl-1-phosphonic acid, 4-Cl-kynurenine, 7-chloro-kynurenic acid, (-)6-phosphonomethyl-decahydroi- soquinoline-3-carboxylic acid, ACPC, aptiganel, besonprodil, BMY-14802, budipine, CGP-37849, CP-101606, conantokin G, CR-3991, CR-2249, CR-3394, delucemine, dexanabinol, dizocilpine, EAA-090, eliprodil, felbamate, fluorofelbamate, FPL-12495, gacyclidine, gavestinel, glycine, harkoseride, HU-211, ibogaine, ipenoxazone, kaitocephalin, ketamine, L-695902, lanicemine, licostinel, ligustizine, midafotel, milnacipran, neboglamine, nebostinel, neramexane, N'-[2-chloro-5-(methylthio)phenyl]-N- -methyl-N-[3-(methylthio)phenyl]-guanidine, N'-[2-chloro-5-(methylthio)phenyl]-N-methyl-N-[3-[(R)-methylsulfinyl]phenyl]-guanidine, neramexane, orphenadrine, remacemide, RGH-896, RG-13484, RG-13579, RG-1103, Ro-25-6981, selfotel, seratrodast, spermidine, spermine, topiramate, traxoprodil, UK-240255, ZD-9379, .alpha.-amino-2-(2-phosphonoethyl)-cyclo- hexanepropanoic acid, alpha.-amino-4-(phosphonomethyl)-benzeneacetic acid, N(1)-(benzyl)cinnamamidine, and 4-benzyl-4-hydroxy-N-(hydroxyphenox- yalkyl)-piperidine, Adamantane, 5-(2-amino-2-carboxyethyl)-4,5-dihydroisoxazole-3-carboxylic acid, Memantine, Cyclohexylbenzene, N-[2-(3,5-dimethyl-1-adamantyl)ethyl] guanidine, N-[2-(3,5-dimethyl-1-adamantyl) ethyl]acetamidine, (+)-SKF38393, and Dopamine.

35. Use of a compound having the formula: 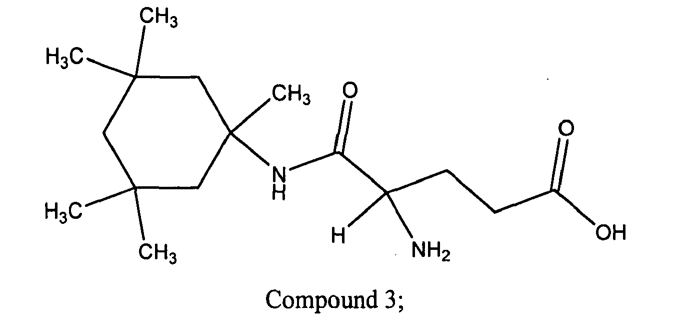 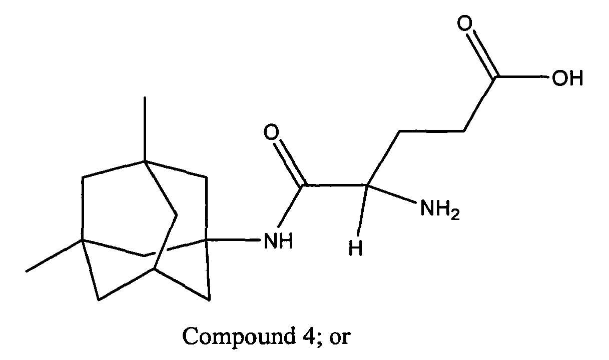 a pharmaceutically acceptable salt or solvate thereof for the manufacture of a medicament for enhancing learning, memory, or cognition.

36. The use of claim 35, wherein the compound is: 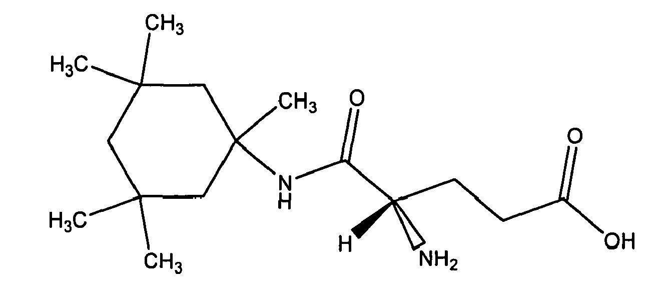 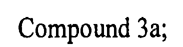 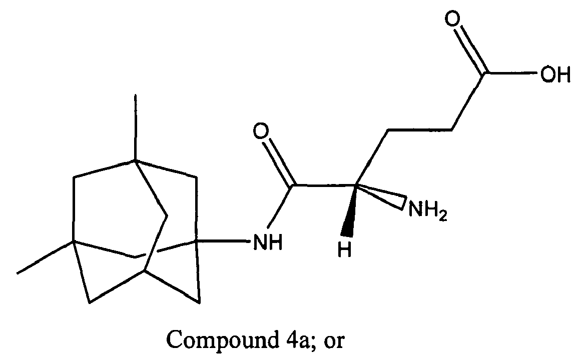 a pharmaceutically acceptable salt or solvate thereof.
